(19) Europäisches Patentamt
European Patent Office
Office européen des brevets

(11) EP 1 446 484 B1

(12) **FASCICULE DE BREVET EUROPEEN**

(45) Date de publication et mention de la délivrance du brevet:
**10.03.2010 Bulletin 2010/10**

(21) Numéro de dépôt: **02796858.5**

(22) Date de dépôt: **21.11.2002**

(51) Int Cl.:
***C12N 15/09*** *(2006.01)* ***C12N 1/21*** *(2006.01)*

(86) Numéro de dépôt international:
**PCT/FR2002/004004**

(87) Numéro de publication internationale:
**WO 2003/044189 (30.05.2003 Gazette 2003/22)**

(54) **SEQUENCE NUCLEOTIDIQUE CODANT POUR UNE PROTEINE D INTERET MODIFIÉ; VECTEUR D EXPRESSION ET PROCEDE D OBTENTION**

NUKLEINSÄURE DIE FÜR EIN GEWÜNSCHTES PROTEIN KODIEREN

NUCLEOTIDE SEQUENCE CODING FOR A MODIFIED PROTEIN OF INTEREST, EXPRESSION VECTOR AND METHOD FOR OBTAINING SAME

(84) Etats contractants désignés:
**AT BE BG CH CY CZ DE DK EE ES FI FR GB GR IE IT LI LU MC NL PT SE SK TR**

(30) Priorité: **21.11.2001 FR 0115081**

(43) Date de publication de la demande:
**18.08.2004 Bulletin 2004/34**

(73) Titulaire: **BIOMERIEUX**
**69280 Marcy-L'Etoile (FR)**

(72) Inventeurs:
- **MALLET, François**
**F-69100 Villeurbanne (FR)**
- **CHEYNET, Valérie**
**F-42410 Verin (FR)**
- **ORIOL, Guy**
**F-42400 Saint Chamond (FR)**
- **ALLARD, Laure**
**F-72000 Le Mans (FR)**
- **NOVELLI-ROUSSEAU, Armelle**
**F-38180 Seyssins (FR)**

(74) Mandataire: **Guerre, Dominique et al**
**Cabinet Germain et Maureau**
**12 Rue Boileau**
**B.P. 6153**
**69466 Lyon Cedex 06 (FR)**

(56) Documents cités:
**WO-A-98/59241 US-A- 5 916 794**

- **GOYAL A ET AL: "INCLUSION OF A FURIN-SENSITIVE SPACER ENHANCES THE CYTOTOXICITY OF RIBOTOXIN RESTRICTION CONTAINING RECOMBINANT SINGLE-CHAIN IMMUNOTOXINS" BIOCHEMICAL JOURNAL, THE BIOCHEMICAL SOCIETY, LONDON, GB, vol. 345, no. PART 2, 15 janvier 2000 (2000-01-15), pages 247-254, XP001008585 ISSN: 0264-6021**
- **HOCULI E ET AL: "GENETIC APPROACH TO FACILITATE PURIFICATION OF RECOMBINANT PROTEINS WITH A NOVEL METAL CHELATE ADSORBENT" BIO/TECHNOLOGY, NATURE PUBLISHING CO. NEW YORK, US, novembre 1988 (1988-11), pages 1321-1325, XP002916185 ISSN: 0733-222X cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne des vecteurs permettant l'expression de protéines modifiées, et leurs applications.

Une protéine modifiée selon l'invention est une protéine, dite d'intérêt, c'est-à-dire une protéine, ou une partie de cette protéine, que l'on cherche à isoler par exemple en diagnostic, à véhiculer par exemple en thérapie, dans la séquence peptidique de laquelle sont rapportées par intercalation et/ou ajout, au moins deux successions de résidus d'acides aminés : une succession d'au moins six résidus lysine et une succession d'au moins six résidus histidine. Dans la suite de la description, on utilisera indifféremment les termes succession et tag pour représenter un groupe de résidus d'acides aminés. Dans les exemples qui suivront la protéine d'intérêt est la glycoprotéine de la capside de VIH-1, p24, mais les objets de l'invention n'y sont bien entendu pas limités.

Selon le document WO-A-98/59241, les auteurs de la présente invention ont démontré que la modification de la séquence peptidique de la protéine p24 de capside du VIH-1, par insertion d'un tag de six résidus lysine, permet d'augmenter considérablement le rendement de couplage de la protéine sur le copolymère AMVE67. On a ainsi pu atteindre une immobilisation de 50 molécules de protéine modifiée par chaîne de copolymère.

L'immobilisation de protéines trouve des applications dans un grand nombre de domaines. Par exemple, en chimiothérapie, l'immobilisation de protéines thérapeutiques permet d'augmenter leur temps de vie dans le sang par limitation des dégradations protéolytiques (Monfardini et al., 1998), mais aussi de cibler passivement des cellules tumorales grâce à l'hyperperméabilité de ces cellules (Duncan et al., 1999). En thérapie génique, on utilise des ligands spécifiques de récepteurs cellulaires et qui sont couplés à des polymères cationiques, pour vectoriser des gènes, permettant un ciblage efficace des cellules à transfecter (Varga et al., 2000).

On sait par tailleurs que le rendement de purification d'une protéine par chromatographie d'affinité pour les ions métalliques immobilisés (IMAC) est fortement élevé quand la protéine est modifiée par apport d'un tag d'au moins six résidus histidine.

Les documents US-A-5,916,794 et E. Hoculi et al., Bio/Technology, Nature Publishing Co New-York, US, Novembre 1988, pp 1321-1325, décrivent des protéines de fusion comprenant une protéine d'intérêt à savoir une endonucléase de restriction pour US-A-5,916,794 et la dihydrofolate réductase pour E. Hoculi et al., et un tag de résidus histidine à l'une ou l'autre des extrémités N- et C-terminales de la protéine d'intérêt. La présence de ce tag permet d'augmenter le rendement d'isolement de la protéine par chromatographie d'affinité pour des métaux immobilisés par chélation.

Selon ces documents, après isolement, le tag histidine est détaché de la protéine d'intérêt, par l'action de la thrombine pour US-A-5,916,794, ou par clivage chimique ou enzymatique, par exemple par action de la carboxypeptidase pour E. Hoculi et al., afin de récupérer, pour utilisation subséquente, la protéine d'intérêt. Cette étape de clivage n'est pas sans risque, car, selon la nature des acides aminés de la protéine d'intérêt et en particulier si elle possède des sites riches en résidus histidine, un clivage non souhaité peut intervenir dans la protéine. De même, les conditions d'un clivage chimique peuvent être préjudiciables à la structure de la protéine d'intérêt.

L'enjeu de l'invention a résidé dans l'obtention d'une protéine modifiée qui, à la fois, soit efficacement purifiable par chromatographie telle que la technique IMAC, soit facilement immobilisable sur un polymère, et possède, une fois purifiée et immobilisée, au moins toutes les propriétés biologiques de la protéine native pour lesquelles la protéine modifiée est utilisée et trouve une application, sans qu'une étape supplémentaire mettant en oeuvre des conditions risquant d'altérer la structure de la protéine, ne soit nécessaire.

Ainsi, un premier objet de l'invention est un procédé de sélection d'un vecteur pour l'expression d'une protéine d'intérêt modifiée, ladite protéine d'intérêt modifiée possédant, après purification et immobilisation, au moins la même activité biologique que la protéine d'intérêt native et étant directement utilisable, ledit vecteur comprenant au moins un gène codant pour ladite protéine d'intérêt, un fragment nucléotidique, dit polyK, codant pour une succession d'au moins six résidus lysine, et un fragment nucléotidique, dit polyH, codant pour une succession d'au moins six résidus histidine.

Au sens de la présente invention, la même activité biologique s'entend en terme qualitatif et en terme quantitatif. La Demanderesse a en effet découvert que l'insertion et/ou l'ajout à la fois d'un tag histidine et d'un tag lysine puis la purification et l'immobilisation de la protéine ainsi modifiée, n'affectaient pas la fonction biologique de la protéine d'intérêt et n'altéraient ni la spécificité, ni la sensibilité de la protéine. Cette observation est surprenante en ce que malgré l'apport de ces deux tags représentant environ au moins 5% de l'ensemble des acides aminés constitutifs d'une protéine, par exemple la protéine p24 de capside du VIH, et malgré l'immobilisation de la protéine ainsi modifiée, celle-ci ne semble pas perdre la conformation qui lui confère son activité. Par directement utilisable, on comprend que la protéine d'intérêt modifiée obtenue peut, après purification et immobilisation, être utilisée comme la protéine d'intérêt, sans étape préalable de traitement pour retirer l'un et/ou l'autre des deux tags histidine et lysine.

L'invention présente un intérêt tout particulier en thérapie génique, où la protéine est couplée à un polymère.

Selon la protéine considérée, et en particulier en fonction de localisation de son ou ses sites d'activité, dans sa séquence peptidique, les tags respectivement de résidus histidine et lysine devront être apportés dans l'une et/ou l'autre des extrémités N- et C-terminales, ou pourront être intercalés entre les épitopes situés dans ladite séquence.

Avantageusement :

les deux tags au moins sont insérés dans, ou ajoutés à, soit l'extrémité N-terminale, soit l'extrémité C-terminale de la protéine ; dans cette configuration, les deux tags peuvent être contigus ou séparés par un espaceur ; ou
un des deux tags est inséré dans, ou ajouté à, l'extrémité N-terminale, et l'autre est inséré dans, ou ajouté à, l'extrémité C-terminale de la protéine.

A cet effet, on a considéré les séquences nucléotidiques suivantes:

- les séquences nucléotidiques dans lesquelles, par rapport audit gène codant pour la protéine d'intérêt, au moins l'un des deux fragments nucléotidiques, polyK ou polyH, est situé sur l'extrémité 5' de la séquence;
- les séquences nucléotidiques dans lesquelles, par rapport audit gène codant pour la protéine d'intérêt, les deux fragments nucléotidiques, polyK ou polyH, sont situé sur l'extrémité 5' de la séquence ; dans cette configuration, soit le fragment nucléotidique polyK est situé entre le fragment nucléotidique polyH et le gène, soit le fragment nucléotidique polyH est situé entre le fragment nucléotidique polyK et le gène ;
- les séquences nucléotidiques dans lesquelles, par rapport audit gène codant pour la protéine d'intérêt, au moins l'un des deux fragments nucléotidiques, polyK ou polyH, est situé sur l'extrémité 5' de la séquence, et l'autre des deux fragments nucléotidiques, polyH ou polyK, est situé sur l'extrémité 3' ; dans cette configuration, soit le fragment nucléotidique polyK est sur l'extrémité 3', et le fragment nucléotidique polyH est sur l'extrémité 5', soit le fragment nucléotidique polyH est sur l'extrémité 3', et le fragment nucléotidique polyK est sur l'extrémité 5' ;
- les séquences nucléotidiques dans lesquelles, par rapport au gène, les deux fragments nucléotidiques, polyK et polyH, sont situés sur l'extrémité 3' de la séquence ; dans cette configuration, soit le fragment nucléotidique polyK est situé entre le fragment nucléotidique polyH et le gène, soit le fragment nucléotidique polyH est situé entre le fragment nucléotidique polyK et le gène ;
- les séquences nucléotidiques, telles que définies ci-dessus et dans lesquelles, entre le gène et au moins l'un des deux fragments polyK et polyH et/ou entre les deux fragments polyK et polyH, est intercalé au moins un fragment nucléotidique codant pour un bras espaceur.

Selon l'invention, une séquence nucléotidique est une séquence dans laquelle le fragment polyK code pour une succession de six résidus lysine, et/ou le fragment polyH code pour une succession de six résidus histidine.
Une séquence nucléotidique codant pour un bras espaceur est avantageusement choisi parmi les séquences nucléotidiques comprenant au moins l'une quelconque des SEQ ID NO :5 à 8. Les séquences SEQ ID NO :9-12 illustrent les séquences peptidiques codées par les séquences nucléotidiques des bras espaceurs SEQ ID NO :5 à 8.
Comme cela sera illustré dans les exemples, dans une application particulière de la détection du VIH-1, la protéine d'intérêt est la p24 de VIH-1, identifiée par SEQ ID NO :13, et la protéine modifiée a une séquence choisie parmi SEC ID NO :14 à 20.
Avant d'exposer en details les objets de l'invention et d'en décrire les caractéristiques et avantages, une définition de certains termes employés dans la description et les revendications est ci-après donnée pour que l'invention et donc l'étendue de la protection soient clairement délimitées.
Une succession ou tag de résidus d'acides aminés est une courte séquence d'acides aminés qui est rapportée dans la séquence peptidique de la protéine native ou originale, en un lieu privilégié, pour permettre à cette succession ou tag d'être exposée de façon pertinente, tout en conservant, voire améliorant, les propriétés biologiques de la protéine native ou originale. En particulier selon l'invention, la présentation du tag de résidus histidine doit être favorable par rapport à une affinité de ce tag pour des ions métalliques, tels qu'utilisés dans la technique de purification dite IMAC (chromatographie par affinité pour ions métalliques immobilisés), celle du tag de résidus lysine doit être favorable par rapport à sa fixation sur une phase d'immobilisation via une interaction covalente entre le tag et des fonctions réactives présentes sur ou dans ladite phase.
Par intercalation ou insertion d'un tag, on comprend que le tag est introduit à l'intérieur de la séquence peptidique de la protéine d'intérêt, entre deux acides aminés. Par ajout d'un tag, on comprend que le tag, on entend que le tag est « rabouté » à la séquence peptidique de la protéine d'intérêt, en l'extrémité N- ou C-terminale de ladite séquence.
En pratique, les protéines modifiées recombinantes décrites, comporteront souvent des acides aminés qui s'intercaleront entre les tags, et/ou entre les tags et la séquence peptidique de la protéine native ou originale, sans pour autant avoir d'incidence sur la spécificité des tags et sur l'activité biologique de la protéine.
Les résidus d'acides aminés appartenant à un tag selon l'invention sont choisis parmi les acides aminés naturels et les acides aminés chimiquement modifiés. La modification chimique apportée à l'acide aminé naturel doit préserver, voire développer, la spécificité du tag vis-à-vis de son rôle dans la fixation. A titre d'exemple, on peut citer le remplacement d'un acide aminé L, par l'acide aminé D correspondant et inversement ; une modification de la chaîne latérale de l'acide aminé : dans le cas de la lysine, il peut s'agir d'une acétylation du groupe aminé de la chaîne latérale ; une modification

des liaisons peptidiques du tag, telles que des liaisons carba, rétro, inverso, rétro-inverso, réduites, méthylène-oxy.

La phase d'immobilisation sur laquelle la fixation de la protéine modifiée est favorisée par l'intermédiaire du tag de résidus lysine peut être un polymère, particulaire ou linéaire, et notamment choisi parmi les homopolymères tels que la polylysine, la polytyrosine ; parmi les copolymères tels que les copolymères d'anhydride maléique, les copolymères de N-vinyl-pyrrolidone, les polysaccharides naturels ou synthétiques, les polynucléotides et les copolymères d'acides aminés comme les enzymes. Des polymères avantageux sont le copolymère N-vinyl-pyrrolidone / N-acryloxysuccinimide, le poly-6-aminoglucose, la peroxydase de raifort (HRP) et la phosphatase alcaline.

La phase d'immobilisation comporte des fonctions réactives qui vont interagir par covalence avec le tag lysine. Ces fonctions réactives sont choisies parmi les fonctions ester, acide, halogénocarbonyle, sulfhydrile, disulfure, époxyde, halogénocarbonyle et aldéhyde.

La phase d'immobilisation peut être fixée, directement ou indirectement, sur un support solide, par adsorption passive ou par covalence.

Ce support solide peut se présenter sous toutes formes appropriées telles qu'une plaque, un cône, une bille, la bille étant éventuellement radioactive, fluorescente, magnétique et/ou conductrice, une barrette, un tube de verre, un puits, une feuille, une puce ou analogues. Le matériau du support est de préférence choisi parmi les polystyrènes, les copolymères styrène-butadiène, les copolymères styrène-butadiène en mélange avec des polystyrènes, des polypropylènes, des polycarbonates, des copolymères polystyrène-acrylonitrile, des copolymères styrène-méthylméthacrylate de méthyle, parmi les fibres synthétiques et naturelles, parmi les polysaccharides et les dérivés de la cellulose, le verre, le silicium et leurs dérivés.

La synthèse d'une séquence nucléotidique décrite précédemment est aisément réalisable par des techniques de routine que l'homme du métier sait mettre en oeuvre.

Lorsque la protéine d'intérêt est la p24 de capside du VIH-1, un vecteur approprié présente une séquence nucléotidique choisie par SEQ ID Nos :1 à 4, de préférence la séquence nucléotidique est SEQ ID NO :1 ou 3.

Les objets de l'invention sont les suivants :

Un procédé de sélection d'un vecteur pour l'expression d'une protéine d'intérêt modifiée, purifiée et immobilisée, ladite protéine d'intérêt modifiée possédant, après purification et immobilisation, au moins la même activité biologique que la protéine d'intérêt native et étant directement utilisable, ledit procédé comprenant les étapes suivantes :

on dispose d'au moins quatre vecteurs comprenant chacun au moins un gène codant pour la protéine d'intérêt, un fragment nucléotidique (polyK) codant pour une succession d'au moins six résidus lysine et un fragment nucléotidique (polyH) codant pour une succession d'au moins six résidus histidine, lesdits vecteurs étant choisis parmi :

i) un vecteur qui comprend de 5' vers 3', le fragment nucléotidique polyH, des sites de clonage pour l'insertion dudit gène d'intérêt et en 3' et le fragment nucléotidique polyK,
ii) un vecteur qui comprend de 5' vers 3', le fragment nucléotidique polyK, des sites de clonage pour l'insertion dudit gène d'intérêt et en 3' le fragment nucléotidique polyH,
iii) un vecteur qui comprend de 5' vers 3', des sites de clonage pour l'insertion dudit gène d'intérêt, le fragment nucléotidique polyK et le fragment nucléotidique polyH, et,
iv) un vecteur qui comprend de 5' vers 3', le fragment nucléotidique polyH, et le fragment nucléotidique polyK et des sites de clonage pour l'insertion dudit gène d'intérêt.

on exprime les séquences nucléotidiques, dans un système d'expression approprié,
on purifie les protéines modifiées, par exemple par chromatographie par affinité aux ions métalliques,
on immobilise les protéines modifiées, purifiées,
on teste l'activité biologique des protéines, modifiées, purifiées et immobilisées, et
on sélectionne, parmi les vecteurs ci-dessus, le vecteur exprimant ladite protéine pour laquelle l'expression est la plus élevée, et/ou pour laquelle le rendement de purification est le plus élevé, et/ou pour laquelle le rendement d'immobilisation est le plus élevé, et/ou pour laquelle l'activité biologique est au moins celle de la protéine d'intérêt native,

ainsi qu'un kit pour l'expression d'une protéine d'intérêt modifiée, telle que précédemment définie, ledit kit comportant quatre vecteurs comprenant chacun une séquence nucléotidique qui comprend au moins un gène codant pour la protéine d'intérêt, un fragment nucléotidique (polyK) codant pour une succession d'au moins six résidus lysine et un fragment nucléotidique (polyH) codant pour une succession d'au moins six résidus histidine, ledit kit consistant en les vecteurs i), ii), iii) et iv), définis ci-dessus.

Selon une variante du procédé de l'invention, celui-ci peut en outre comprendre les étapes suivantes :

Après l'étape de purification, on peut sélectionner la ou les protéines pour lesquelles le rendement de purification est le plus élevé, et/ou

Après l'étape d'immobilisation, on peut sélectionner la ou les protéines pour lesquelles le rendement d'immobilisation est le plus élevé.

Ce procédé permet d'obtenir une protéine d'intérêt modifiée, purifiée et immobilisée, dans laquelle la position des tags histidine et lysine est optimale du point de vue de l'activité biologique de la protéine modifiée.

Une protéine d'intérêt modifiée ainsi obtenue est facilement purifiable, immobilisable et directement utilisable, après purification et immobilisation, ces étapes étant réalisées avec des rendements très élevés.

Les caractéristiques et avantages des différents objets de l'invention sont ci-après illustrés, à l'appui des Exemples 1 à 6 et des Figures 1 à 6, selon lesquelles :

La Figure 1 illustre la protéine native p24 et les différentes protéines modifiées, telles qu'exprimées par des vecteurs selon la présente invention.

La Figure 2 illustre l'analyse sur gel de polyacrylamide de l'expression et de la purification des protéines recombinantes ; Figure 2A montre le taux d'expression des différentes protéines avant et après induction à l'IPTG, ; Figure 2B montre le taux de pureté des différentes protéines après purification par chélation métallique pour les ions $Zn^{2+}$ ; Figure 2C montre la reconnaissance des protéines purifiées par un anticorps polyclonal après transfert par Western Blot sur membrane de nitrocellulose.

La Figure 3 illustre les caractéristiques physico-chimiques des sept protéines recombinantes décrites en Figure 1, et plus particulièrement le nombre d'acides aminés les constituant et leur masse moléculaire déterminée par spectrométrie de masse et comparée à la masse moléculaire théorique.

La Figure 4 représente un histogramme montrant l'efficacité de couplage en pourcentage des sept protéines recombinantes sur le polymère d'AMVE67.

La Figure 5 illustre la comparaison des réactivités biologiques des conjugués RH24K-AMVE67 et RK24H-AMVE67 en phase de capture d'anticorps monoclonaux en fonction de la position de l'épitope reconnu par l'anticorps.

La Figure 6 illustre la structure des vecteurs d'expression pMK permettant d'obtenir des protéines modifiées selon l'invention. La Figure 6A schématise la structure d'un vecteur, et la Figure 6B montre quatre configurations de vecteurs permettant d'obtenir les protéines modifiées suivantes : RH24K, R24KH, RK24H et RHK24.

## Exemple 1: Ensemble de constructions permettant l'obtention de protéines doublement taggées

Schématiquement, les vecteurs permettant l'expression des protéines recombinantes taggées ont été générés à partir du vecteur d'expression pMR24 obtenu par ligation du fragment NcoI-XbaI du pMH24 (Cheynet *et al,* 1993) contenant le gène de la p24 avec le fragment NcoI-XbaI du pMR-T7 (WO-98/45449, Arnaud et al, 1997) contenant l'ensemble des séquences régulant la réplication du plasmide ainsi que les éléments permettant l'expression du gène inséré. Des adaptateurs oligonucléotidiques adéquats amenant les informations codantes relatives aux tags lysine et/ou histidine ont été insérés entre ClaI et NcoI en 5' et SmaI et XbaI en 3', pour obtenir une séquence nucléotidique selon l'invention. La portion du gène de la p24 codant pour le polypeptide commençant à l'acide aminé 3 (valine) et se terminant à l'acide aminé 224 (proline) est conservée dans toutes les constructions.

Les sept séquences nucléotidiques insérées ont été conçues comme suit : toutes possèdent une séquence nucléotidique codant pour une succession (ou tag) de 6 résidus histidine, devant permettre une purification efficace de la protéine par affinité pour les ions métalliques (IMAC pour chromatographie par affinité sur ions métalliques immobilisés), et cinq d'entre elles présentent une séquence codant pour une succession (ou tag) de six lysines, afin de permettre un couplage covalent de la protéine sur le polymère.

Les protéines modifiées recombinantes obtenues sont les suivantes :

- RH24 codée par le plasmide pRH24, possède un tag de 6 résidus histidine en position N-terminale, illustrée par SEQ ID NO :14
- R24H, codée par le plasmide pRH24 et pR24H, présente un tag de 6 résidus histidine en position C-terminale, illustrée par SEQ ID NO :15 ;
- RH24K, codée par le plasmide pRH24K, présente un tag de 6 résidus histidine en position N-terminale et un tag de 6 résidus lysine en position C-terminale, illustrée par SEQ ID NO :16 ;
- RK24H, codée par le plasmide pRK24H, présente un tag de 6 résidus histidine en position C-terminale et un tag de 6 résidus lysine en position N-terminale, illustrée par SEQ ID NO :17 ;
- R24KH, codée par le plasmide pR24KH, présente un tag de 6 résidus histidine et un tag de 6 résidus lysine ; tous deux sont en position C-terminale et sont contigüs, illustrée par SEQ ID NO :18 ;
- R24KsH, codée par le plasmide pR24KsH, présente un tag de 6 résidus lysine et un tag de 6 résidus histidine ;

tous deux sont en position C-terminale et sont séparés par une séquence espaceur, illustrée par SEQ ID NO :19 ;

- RHsK24, codée par le plasmide pRHsK24, présente un tag de 6 résidus histidine et un tag de 6 résidus lysine ; tous deux sont en position N-terminale et sont séparés par une séquence espaceur, illustrée par SEQ ID NO :20 ;

La séquence espaceur des protéines recombinantes R24KsH et RHsK24 est représentée par « s » et est constituée par une succession de quatre résidus glycine et un résidu sérine, qui peut être répétée plusieurs fois.

La Figure 1A décrit la séquence peptidique de la protéine p24 native de capside du VIH-1, isolée de la souche HXB2. Le fragment peptidique 3-224 représente la séquence conservée dans toutes les protéines recombinantes.

La Figure 1B illustre la structure des sept protéines recombinantes, ci-dessus, la séquence peptidique conservée étant représentée par une boîte blanche, le tag de 6 résidus histidine étant représenté par une boîte grise, le tag de 6 résidus lysine étant représenté par une boîte noire ; les résidus d'acide aminé précisément indiqués sont des acides aminés spécifiques, hors des trois boîtes précédentes et de la séquence espaceur, pouvant varier d'une protéine recombinante à une autre.

**Exemple 2 : Obtention de protéines recombinantes taggées $H_6$ et $K_6$**

Des bactéries compétentes *E. coli* souche XL1 ont été transformées par les sept plasmides obtenus à l'Exemple 1, et l'induction de l'expression des protéines est réalisée par addition d'isopropyl-β-D-thiogalactopyranoside (IPTG), comme précédemment décrit (Cheynet *et al,* 1993, Arnaud *et al,* 1997). Les protéines sont extraites, après sonication du culot bactérien, en tampon 50 mM Tris pH 8,0, 1 mM EDTA, 10 mM $MgCl_2$, 100 mM NaCl en présence d'anti-protéases (leupeptine 10 μg/μl et aprotinine 1,25 μg/μl), puis purifiées par IMAC. Les purifications ont été réalisées sur un gel de Sépharose activé avec des ions zinc. Les protéines recombinantes comprenant un tag de 6 résidus histidine sont chélatées par les ions métalliques. Le système chromatographique utilisé est une FPLC (Äkta Explorer, Pharmacia Biotech). La boucle de chargement est de 2 ml. Les purifications se font par injection de protéine diluée au 1/2 dans le tampon de lavage qui est un tampon phosphate 67 mM - NaCl 0,5 M pH7,8. Les protéines d'intérêt sont éluées spécifiquement à pH 4,7 environ par élaboration d'un gradient de pH à l'aide de tampons acétate d'ammonium pH 6,0 et pH 3,0. Les différentes fractions de purification sont collectées. 10 μl de chacune de ces fractions sont déposés sur papier Whatman 3MM Chr puis colorés au bleu de Coomassie. Les fractions (protéines non retenues - protéine purifiée) sont ensuite mises à migrer sur gels d'acrylamide 12% après réduction au β-Me et chauffage 10 minutes à 95°C, puis colorées au bleu de Coomassie. Les fractions les plus concentrées en protéine d'intérêt sont alors rassemblées puis dialysées en cassette de dialyse Slide-A-Lyzer MWCO 10000 PIERCE 1 heure puis 1 nuit à +4°C contre un tampon Phosphate 50 mM pH 7,8. Les concentrations en protéines sont alors définies par un dosage colorimétrique Bradford Coomassie Plus Assay (PIERCE).

Les extraits protéiques bactériens et les protéines purifiées sont mis à migrer sur gels d'acrylamide 12% après réduction au β-mercapto-éthanol et chauffage 10 minutes à 95°C, puis colorés au bleu de Coomassie. Pour les protéines purifiées, un gel réalisé en parallèle est transféré par Western Blot sur membrane de nitrocellulose (Hybond C extra, Amersham Life Science). Les sites non spécifiques de la membrane sont ensuite saturés en tampon Tris salin (TBS pour Tris Buffer Salin) Tween 0,1% additionné de 5% de lait. Après 3 lavages en TBS-T, la membrane est mise à incuber 2 heures à température ambiante en présence de l'anticorps polyclonal de lapin biotinylé anti-p24 dilué au 1/10 $000^{\text{ème}}$ en tampon TBS-T + 5% lait. Après 3 lavages en TBS-T, la membrane est mise à incuber 1 heure à température ambiante en présence de streptavidine - peroxydase (Jackson ImmunoResearch) à 0,5 g/l diluée au 1/3 $000^{\text{ème}}$ en tampon TBS-T + 5% lait. Trois lavages en TBS-T sont réalisés avant la révélation par chimiluminescence ECL+ (Amersham Pharmacia Biotech, RPN2132). Une autoradiographie de 15 secondes en chambre noire est réalisée sur film Kodak Biomax MR.

La Figure 2 illustre l'analyse sur gel de polyacrylamide de l'expression et de la purification des protéines recombinantes comme suit.

La Figure 2A présente le résultat d'une analyse sur gel d'acrylamide 12% coloré au bleu de Coomassie des fractions non induites (-) et induites (+) à l'IPTG 1 mM pendant 3 heures à 37°C des sept protéines recombinantes, avec un dépôt de 5 μl / puits d'échantillon brut. La protéine produite est indiquée par une flèche (>).

La Figure 2B présente le résultat de l'analyse sur gel d'acrylamide 12% coloré au bleu de Coomassie des sept protéines recombinantes, après leur purification par chélation pour les ions métalliques $Zn^{2+}$, avec un dépôt de 3 μg / puits.

La Figure 2C représente le résultat du transfert des protéines sur membrane de Nitrocellulose par Western Blot après migration sur gel d'acrylamide 12%. La reconnaissance est effectuée par un anticorps polyclonal de lapin biotinylé dilué au $1/10000^{\text{ème}}$ et révélation par chimiluminescence ECL+, après exposition du film d'autoradiographie pendant 15 secondes. Le dépôt était de 0,127 μg / puits.

L'analyse de l'expression (Figure 2A) montre que, pour 6 des 7 protéines attendues, les protéines d'intérêts représentent environ 20 à 30% des protéines totales produites par la bactérie *E. coli* après induction (+), indépendamment de l'introduction du tag Lys-6 (par comparaison de RH24K et RH24, de RK24H et R24H) et des positions respectives des tags His-6 et Lys-6 (par comparaison de RH24K, RK24H, R24KH et R24KsH). La protéine RHsK24 présente quant

à elle, un faible niveau d'expression, avec moins de 5% de la quantité des protéines totales.

Au final, on obtient des quantités similaires des protéines recombinantes RH24, R24H, RH24K, RK24H, R24KH et R24KsH, à savoir entre 2 et 5 mg par gramme de biomasse pour une condition de culture et d'extraction donnée, et seulement 0,4 mg de RHsK24, en accord avec son faible niveau d'expression. On observe qu'en optimisant les conditions de cultures telles que le volume de culture et l'étape d'extraction, des rendements de 9 à 16 mg par gramme de biomasse ont pu être obtenus pour la RH24 et la RH24K.

Le résultat de l'étape de purification des protéines est représentée sur la Figure 2B, et on observe que la pureté sur gel après coloration au bleu de Coomassie est supérieure à 95%. Une reconnaissance sur membrane de nitrocellulose par un anticorps polyclonal de lapin anti-p24 fait apparaître selon la Figure 2C que les protéines obtenues correspondent bien à celles attendues. Elles migrent à une taille de 27 kDa environ, ce qui est en accord avec la valeur attendue. Certaines protéines présentent de faibles bandes supplémentaires de masse inférieure et de très faible intensité.

**Exemple 3 : Caractérisation des protéines recombinantes**

Les protéines purifiées sont ensuite plus précisément caractérisées par spectrométrie de masse couplée à une chromatographie liquide (LC/ESI/MS). Les analyses ont été réalisées sur un spectromètre de masse simple quadripôle API 100, des pompes 140B et un détecteur 785A (Perkin Elmer). Les chromatographies liquides en phase inverse ont été réalisées sur une colonne C4 (Vydac Ref 214PT5115, taille des particules 5 μm). Les tampons d'élution sont pour le solvant A : acide formique dans de l'eau 0, 1 % (v/v) et pour le solvant B : acide formique dans une solution eau / acétonitrile (5 : 95 v/v). Un gradient de 40 à 60% de B a été utilisé.

Pour chaque protéine recombinante, sont indiqués, sur la Figure 3, le nombre d'acides aminés, les masses moléculaires (MM) théoriques ([a]) déterminées grâce à l'utilisation du logiciel Mac Vector Version 6.5.3 et les masses moléculaires expérimentales ([b]) déterminée par spectrométrie de masse couplée à une chromatographie liquide (LC/ESI/MS).

Les résultats montrent que les masses moléculaires déterminées par spectrométrie de masses sont conformes à celles attendues pour les protéines RH24, RH24K, RK24H et RHsK24, et que donc les protéines utilisées correspondent à celles déduites de la traduction du gène modifié. Les protéines R24KsH R24KH R24H présentent respectivement un défaut de masse de 119, 121 et 123 Da, correspondant probablement à la perte de l'isoleucine carboxy-terminale. Ceci n'affecte aucun des deux tags.

**Exemple 4 : Obtention de conjugués protéines-polymère**

L'efficacité de couplage de ces protéines diversement taggées sur des copolymères d'anhydride maléique a été testée. L'immobilisation covalente de protéines sur des polymères se fait par l'établissement d'une liaison amide covalente entre les groupements anhydride du polymère et les amines primaires présentes sur les chaînes latérales des résidus lysine comme illustré dans le schéma ci-dessous. Cependant, le polymère n'étant pas hydrosoluble, il est nécessaire de le dissoudre dans du DMSO (diméthylsulfoxyde) anhydre préalablement à la réaction de couplage réalisée en milieu aqueux à 95%.

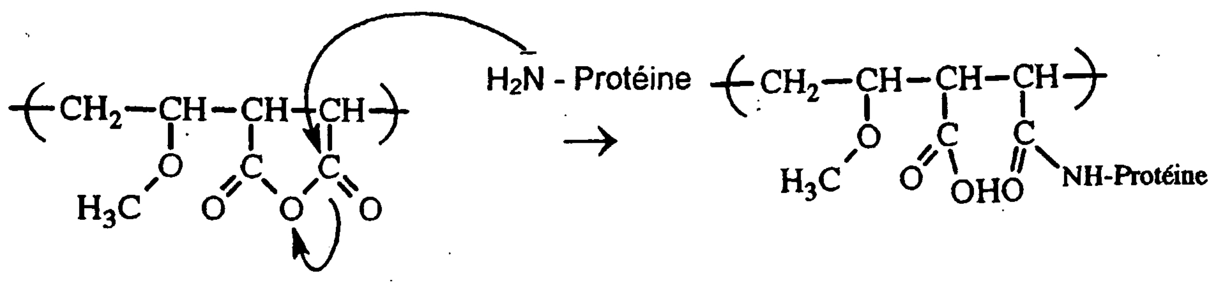

Conditions opératoires :

Tampons de couplage : Phosphate 50 mM pH 7,8,

Polymère : peser 2 mg de copolymère AMVE 67 000 (Polysciences INC lot N°427393) et dissoudre doucement dans 2 ml de DMSO anhydre.

Protéine : décongeler doucement dans la glace, la quantité requise pour le couplage
Protocole de couplage :
100 ou 36 μg de protéines
5 μl de polymère à 1 g/l en DMSO ($7{,}46.10^{-11}$ moles)
qsp 105 μl tampon phosphate 50 mM pH 7,8

La réaction de couplage covalent est réalisée spontanément par incubation pendant 3 heures à 37°C sur agitateur thermique.

Les conjugués sont ensuite caractérisés comme suit.

Les échantillons sont filtrés dans des tubes Ultrafree Millex HV 0,45 μm (Millipore) puis analysés par chromatographie d'exclusion stérique sur colonne Shodex Protein KW 803. Le système chromatographique est une HPLC Kontron comprenant une pompe 422, un injecteur automatique 465 et une DAD (Diode Array Detector). L'élution est réalisée en tampon phosphate 0,1 M pH 6,8 + 0,5% SDS (m/m) avec un flux de 0,5 ml/min. La détection est réalisée par mesure de l'absorbance à 280 (à la concentration utilisée, le polymère n'absorbe pas).

Le ratio de l'air du pic correspondant à la protéine couplée au polymère *versus* la somme des deux pics correspondants aux protéines coupées et non coupées (c'est-à-dire la quantité totale des protéines impliquées dans la réaction) donne la valeur du rendement de couplage (Y).

$$\frac{(\text{Aire du pic de conjugué protéine / polymère})_{280\ nm} \times 100}{(\text{Aire du pic de conjugué protéine/polymère})_{280\ nm} + (\text{Aire du pic de protéine libre})_{280\ nm}}$$

Le nombre de protéines par chaîne de polymère est défini par la relation suivante : N = n.Y/n' ou n et n' représentent respectivement le nombre de moles de protéines et le nombre de chaînes de polymère dans le milieu réactionnel.

Les données de la Figure 4 illustrent les rendement de couplage en pourcentage des sept protéines recombinantes RH24, R24H, RH24K, RK24H, R24KH, R24KsH et RHsK24 dérivées de la protéine de capside p24 du VIH-1 sur le copolymère d'AMVE67 en tampon phosphate 50 mM pH 7,8.

Les concentrations utilisées sont les suivantes [protéines] = 0,95 g/l ($3{,}56.10^{-9}$ mol), [AMVE67] = 0,048 g/l ($7{,}46.10^{-11}$ mol).

□ représente les protéines ne contenant qu'un tag de 6 résidus histidine, ■ représente les protéines avec un tag de 6 résidus histidine opposé au tag de 6 résidus lysine, ■ représente les protéines avec des tags de 6 résidus histidine et 6 résidus lysine contigus. Les expériences ont été réalisées 3 fois, les valeurs indiquées correspondent à la moyenne plus un écart type.

En l'absence de résidus lysine, les rendements de couplage sont compris entre 10 et 30%. Ils sont supérieurs à 95% lorsque le tag de 6 résidus lysine est présent sur la protéine. La présence d'un tag de 6 résidus lysine permet donc d'améliorer considérablement l'efficacité de couplage (par comparaison de RK24H, R24KH, R24KsH, RHsK24 avec RH24 et R24H), indépendamment de sa position N- ou C-terminale (comparaison de RK24H avec RH24K, RHsK24 avec R24KsH), en opposition ou adjacent au tag de 6 résidus histidine (comparaison de RH24K et RK24H avec R24KH, R24KsH, RHsK24).

**Exemple 5: Bioréactivité des protéines ainsi couplées**

L'amélioration du rendement de couplage des protéines Lys-6 sur le copolymère AMVE67 suggère que la réaction de couplage est régio-sélective, à savoir qu'elle implique le tag de résidus lysine.

On a évalué la réactivité biologique des conjugués en fonction de la position N- ou C-terminale du tag et de la position N- ou C-terminale de l'épitope reconnu par l'anticorps monoclonal. Deux protéines ont été retenues pour cette étude, RH24K et RK24H, présentant respectivement un tag de six résidus lysine en position C-terminale et N-terminale, et opposé au tag de six résidus histidine.

Le protocole d'ELISA a été réalisé comme suit : 100 μl / puits de conjugué protéines-polymère dilué à 0,25 μg / ml en tampon PBS sont immobilisés en fond de microplaque 96 puits (Nunc Immuno[a] Plate Maxisorp[a] surface) par incubation d'une nuit à température ambiante. Les sites non spécifiques sont alors saturés pendant 2 heures à 37°C par 200 μl / puits d'une solution de PBS-1% Régilait™ (p/v). Les puits sont alors lavés 3 fois en PBS-tween 0,05%. Les anticorps monoclonaux dilués à la dilution appropriée en tampon PBS-tween 0,05%-0,2% Régilait™ sont alors incubés pendant 1 heure à 37°C. Après 3 lavages en PBS-tween 0,05%, le conjugué anti-souris marqué à la peroxydase (Jackson ImmunoResearch) dilué au 1/2 000 en PBS-tween 0,05%-1% Régilait™ est incubé 1 heure à 37°C. Trois lavages en

PBS-tween 0,05% sont réalisés avant la révélation au cours de laquelle 100 μl d'une solution contenant une pastille d'OPD 30 mg diluée dans 10 ml de tampon substrat OPD (Sanofi pasteur) sont incubés 10 min à l'obscurité à température ambiante. La réaction est alors bloquée par addition de 100μl / puits de $H_2SO_4$ 1 N, puis les valeur d'absorbance sont lues au spectrophotomètre à 492 nm.

Les données de la Figure 5 sont comme suit :

Le Tableau indique le signal obtenu en ELISA en coating de conjugué protéine-polymère
[a] Protéines RH24K et RK24H couplées au polymère AMVE67.
[b] Position de l'épitope reconnue par l'anticorps monoclonal.
[c] La détection a été réalisée en utilisant un anticorps monoclonal.
[d] Ratio déterminé à partir de la DO de l'échantillon testé ($DO_{ET}$) et de la DO du conjugué de référence RH24K-AMVE67 ($DO_{Ref}$).

Les résultats montrent que le signal ELISA est meilleur lorsque le tag se trouve en position opposé à l'épitope reconnu par un anticorps monoclonal. Ainsi, anticorps monoclonal reconnaissant un épitope localisé en position N-terminale (AcM 15F8) présente un signal 1,3 fois plus élevé pour une protéine immobilisée via sa région C-terminale (RH24K) que pour une protéine immobilisée via sa région N-terminale (RK24H). Inversement, un anticorps reconnaissant un épitope localisé en position C-terminale présente un signal 8,3 fois (AcM 23A5) et 2,25 fois (AcM 3D8) plus élevé lorsque la protéine est immobilisée via sa région N-terminale (RK24H) que lorsque celle-ci est immobilisée via sa région C-terminale (RH24K).

**Exemple 6: préparation d'un kit de vecteurs d'expression de protéines modifiées**

Compte tenu des capacités d'expression, de purification et de couplage orienté présentées par les différentes protéines doublement taggées, dérivées du modèle p24, on a réalisé des vecteurs d'expression permettant l'insertion d' un gène d'intérêt pour lequel les trois propriétés seraient requises. Ces vecteurs combinent des séquences codant pour un tag de six résidus histidine permettant une purification efficace par chélation pour les ions métalliques et un tag de six résidus lysine pour une immobilisation covalente orientée. En fonction de l'application et/ou de restrictions imposées par la position du site actif de la protéine, les vecteurs d'expression proposés présentent différentes combinaisons possibles.

Le vecteur pMK81 dérive du vecteur d'expression pH24K par clivage avec NcoI et SmaI, puis par ligation à la séquence du polyKinker NcoI-SmaI. Le vecteur pMK81 contient en 5' une trame de lecture codant pour un tag His-6, des sites de clonages uniques pour l'insertion de gènes codant pour des protéines d'intérêt, et en 3' une trame de lecture codant pour un tag Lys-6. Sa taille est de 4935 bp.

Le vecteur pMK82 dérive du vecteur d'expression p24KH par clivage avec NcoI et SmaI, puis par ligation à la séquence du polyKinker NcoI-SmaI. Le vecteur pMK82 contient en 5' un codon initiateur de la traduction, des sites de clonages uniques pour l'insertion de gènes codant pour des protéines d'intérêt, et en 3' une trame de lecture codant pour un tag double Lys-6 His-6. Sa taille est de 4921 bp.

Le vecteur pMK83 dérive du vecteur d'expression pK24H par clivage avec NcoI et XhoI, puis par ligation à la séquence du polyKinker NcoI-XhoI. Durant la construction, le site XhoI a éliminé. Les oligonucléotides double brin ont été obtenus par hybridation de chaque brin en tampon 50 mM NaCl, 6 mM Tris/HCl, pH 7.5, 8 mM $MgCl_2$), par chauffage 5 minutes à 65°C, et refroidissement lent à température ambiante. Le vecteur pMK83 contient en 5' une trame de lecture codant pour un tag Lys-6, des sites de clonages uniques pour l'insertion de gènes codant pour des protéines d'intérêt, et en 3' une trame de lecture codant pour un tag His-6. Sa taille est de 4945 bp.

Le vecteur pMK84 dérive du vecteur d'expression pHK24 par clivage avec NcoI et SmaI, puis par ligation à la séquence du polyKinker NcoI-SmaI. Le vecteur pMK84 contient en 5' une trame de lecture codant pour un tag double His-6 Lys-6, des sites de clonages uniques pour l'insertion de gnes codant pour des protéines d'intérêt, et en 3' un codon terminateur de la traduction. Sa taille est de 4951 bp.

Les caractéristiques des vecteurs représentés sur la Figure 6 sont comme suit :

La Figure 6A représente la structure des vecteurs d'expression pMK. P*tac,* promoteur *tac* (boite noire) ; RBS1-MC-RBS2, minicistron flanqué par 2 sites de fixation des ribosomes (RBS) (flèche blanche), MCS, site de clonage multiple (boite grise) ; rrnB T1 T2, terminateurs de transcription forts (boite en pointillés) ; *bla,* gène conférant la résistance à l'ampicilline (flèche noire); pMB1 ori / M13 ori, origines de réplication (boite blanche fine) ; $lacI^q$, gène codant pour le répresseur $lacI^q$ (flèche hachurée). Les sites de restriction ClaI et XbaI flanquant le MCS sont soulignés.
La Figure 6B représente les séquences des vecteurs d'expression pMK81, pMK82, pMK83 et pMK84 entourant le minicistron (RBS1 et RBS2 soulignés, la courte trame de lecture ouverte en petit caractères), les codons d'initiation et de terminaison (caractère gras) et les sites de restriction du site de clonage multiple. Les séquences en acides

aminés correspondant aux régions amino- et carboxy-terminales des protéines recombinantes, incluant les tags sont indiquées.

BIBLIOGRAPHIE

Monfardini C. and F. M. Veronese. 1998 Stabilization of Substances in Circulation (review) Bioconjugate Chem. 9: 418-450.

Duncan R. 1999 Polymer conjugates for tumour targeting and intracytoplasmic delivery. The EPR effect as a common gateway? Pharmaceutical Science & Technology Today 2(11): 441-449.

Varga C.M. , Wickham T.J., and D.A. Lauffenburger. 2000 Receptor-mediated targeting of gene delivery vectors: Insights from molecular mechanisms for improved vehicle design (Review).Biotechnology and Bioengineering 70 (6): 593-605

Ladavière C., T. Delair, A. Domard, A. Novelli-Rousseau, B. Mandrand et F. Mallet. 1998. Covalent immobilization of proteins onto (maleic anhydride-a/t-methyl vinyl ether) copolymers: enhanced immobilization of recombinant proteins. Bioconjug Chem 9(6):655-661.

Laure Allard, Valérie Cheynet, Guy Oriol, Laurent Véron, Françoise Merlier, Gérald Scrémin, Bernard Mandrand, Thierry Delair and Franois Mallet 2001 Mechanisms Leading to an Oriented Immobilization of Recombinant Proteins Derived from the p24 Capsid of HIV-1 onto Copolymers. Bioconjug Chem in press

Cheynet, V., B. Verrier, and F. Mallet. 1993. Overexpression of HIV-1 proteins in Escherichia coli by a modified expression vector and their one-step purification. Prot Express Purif 4:367-372.

Berthet-Colominas C., S. Monaco, A.Novelli, G. Sibaï, F. Mallet and S. Cusack. 1999. Head-to-tail dimers and interdomain flexibility revealed by the crystal structure of HIV-1 capsid protein (p24) complexed with a monoclonal antibody Fab. EMBO 18(5): 1124-1136.

Arnaud N., V. Cheynet, G. Oriol, B. Mandrand and F. Mallet. 1997. Construction and expression of a modular gene encoding bacteriophage T7 RNA polymerase. Gene 199(1-2):19-156.

Ganachaud F., Mouterde G, Delair T, Elaïssari A. and Pichot C. 1995 Preparation and characterization of cationic polystyrene latex particles of different aminated surface charges. Polymers for Advanced Technologies 6: 480-488.


## Revendications

**1.** Procédé de sélection d'un vecteur pour l'expression d'une protéine d'intérêt modifiée, purifiée et immobilisée, ladite protéine d'intérêt modifiée possédant, après purification et immobilisation, au moins la même activité biologique que la protéine d'intérêt native et étant directement utilisable, ledit procédé étant **caractérisé en ce qu'**il comprend les étapes suivantes :

on dispose d'au moins quatre vecteurs comprenant chacun au moins un gène codant pour la protéine d'intérêt, un fragment nucléotidique (polyK) codant pour une succession d'au moins six résidus lysine et un fragment nucléotidique (polyH) codant pour une succession d'au moins six résidus histidine, lesdits vecteurs étant choisis parmi :

i) un vecteur qui comprend de 5' vers 3', le fragment nucléotidique polyH, des sites de clonage pour l'insertion dudit gène d'intérêt et en 3' et le fragment nucléotidique polyK,
ii) un vecteur qui comprend de 5' vers 3', le fragment nucléotidique polyK, des sites de clonage pour l'insertion dudit gène d'intérêt et en 3' le fragment nucléotidique polyH,
iii) un vecteur qui comprend de 5' vers 3', des sites de clonage pour l'insertion dudit gène d'intérêt, le fragment nucléotidique polyK et le fragment nucléotidique polyH, et,
iv) un vecteur qui comprend de 5' vers 3', le fragment nucléotidique polyH, et le fragment nucléotidique polyK et des sites de clonage pour l'insertion dudit gène d'intérêt.

on exprime les séquences nucléotidiques, dans un système d'expression approprié,
on purifie les protéines modifiées,
on immobilise les protéines modifiées, purifiées,
on teste l'activité biologique des protéines, modifiées, purifiées et immobilisées, et
on sélectionne, parmi les vecteurs ci-dessus, le vecteur exprimant ladite protéine pour laquelle l'expression est la plus élevée, et/ou pour laquelle le rendement de purification est le plus élevé, et/ou pour laquelle le rendement d'immobilisation est le plus élevé, et/ou pour laquelle l'activité biologique est au moins celle de la protéine d'intérêt native.

**2.** Procédé selon la revendication 1, **caractérisé en ce que** le fragment nucléotidique polyK code pour une succession de six résidus lysine et le fragment nucléotidique polyH code pour une succession de six résidus histidine.

**3.** Procédé selon la revendication 1 ou 2, **caractérisé en ce que** les fragments nucléotidiques polyH et polyK du vecteur identifié en (iii) sont contiguës.

**4.** Procédé selon l'une quelconque des revendications 1 à 3, **caractérisé en ce que** les fragments nucléotidiques polyH et polyK du vecteur identifié en (iv) sont contiguës.

**5.** Procédé selon l'une quelconque des revendications 1, 2 et 4, **caractérisé en ce que** les fragments nucléotidiques polyH et polyK du vecteur identifié en (iii) sont séparés par une séquence nucléotidique codant pour un bras espaceur choisie parmi SEQ ID NO : 5 à 8.

**6.** Procédé selon l'une quelconque des revendications 1, 2 et 3, **caractérisé en ce que** les fragments nucléotidiques polyH et polyK du vecteur identifié en (iv) sont séparés par une séquence nucléotidique codant pour un bras espaceur choisie parmi SEQ ID NO : 5 à 8.

**7.** Procédé selon l'une quelconque des revendications 1 à 6, **caractérisé en ce que** la protéine d'intérêt est la protéine de VIH-1, p24.

**8.** Procédé selon la revendication 7, **caractérisé en ce que** la protéine p24 est identifiée par SEQ ID NO : 13 et la protéine modifiée a une séquence choisie parmi SEQ ID NO : 14 à 20.

**9.** Procédé selon l'une quelconque des revendications 1 à 8, **caractérisé en ce qu'**on purifie la protéine modifiée, par chromatographie par affinité aux ions métalliques.

**10.** Procédé selon l'une quelconque des revendications 1 à 9, **caractérisé en ce qu'**on immobilise la protéine modifiée sur un polymère linéaire ou particulaire.

**11.** Kit pour l'expression d'une protéine d'intérêt modifiée, purifiée et immobilisée, ladite protéine d'intérêt modifiée possédant, après purification et immobilisation, au moins la même activité biologique que la protéine d'intérêt native et étant directement utilisable, ledit kit comportant quatre vecteurs comprenant chacun une séquence nucléotidique qui comprend au moins un gène codant pour la protéine d'intérêt, un fragment nucléotidique (polyK) codant pour une succession d'au moins six résidus lysine et un fragment nucléotidique (polyH) codant pour une succession d'au moins six résidus histidine, ledit kit consistant en les vecteurs suivants :

i) un vecteur qui comprend de 5' vers 3', le fragment nucléotidique polyH, des sites de clonage pour l'insertion dudit gène d'intérêt et en 3' et le fragment nucléotidique polyK,
ii) un vecteur qui comprend de 5' vers 3', le fragment nucléotidique polyK, des sites de clonage pour l'insertion dudit gène d'intérêt et en 3' le fragment nucléotidique polyH,
iii) un vecteur qui comprend de 5' vers 3', des sites de clonage pour l'insertion dudit gène d'intérêt, le fragment nucléotidique polyK et le fragment nucléotidique polyH, et,
iv) un vecteur qui comprend de 5' vers 3', le fragment nucléotidique polyH, et le fragment nucléotidique polyK et des sites de clonage pour l'insertion dudit gène d'intérêt.

**12.** Kit selon la revendication 11, **caractérisé en ce que** le fragment nucléotidique polyK code pour une succession de six résidus lysine et le fragment nucléotidique polyH code pour une succession de six résidus histidine.

**13.** Kit selon la revendication 11 ou 12, **caractérisé en ce que** les fragments nucléotidiques polyH et polyK du vecteur identifié en (iii) sont contiguës.

**14.** Kit selon l'une quelconque des revendications 11 à 13, **caractérisé en ce que** les fragments nucléotidiques polyH et polyk du vecteur identifié en (iv) sont contiguës.

**15.** Kit selon l'une quelconque des revendications 11, 12 et 14, **caractérisé en ce que** les fragments nucléotidiques polyH et polyK du vecteur identifié en (iii) sont séparés par une séquence nucléotidique codant pour un bras espaceur choisie parmi SEQ ID NO : 5 à 8.

**16.** Kit selon l'une quelconque des revendications 11, 12 et 13, **caractérisé en ce que** les fragments nucléotidiques polyH et polyK du vecteur identifié en (iv) sont séparés par une séquence nucléotidique codant pour un bras espaceur choisie parmi SEQ ID NO : 5 à 8.

**17.** Kit selon l'une quelconque des revendications 11 à 16, **caractérisé en ce que** la protéine d'intérêt est la protéine de VIH-1, p24.

**18.** Kit selon la revendication 17, **caractérisé en ce que** la protéine p24 est identifiée par SEQ ID NO : 13 et la protéine modifiée a une séquence choisie parmi SEQ ID NO : 14 à 20.

## Claims

**1.** A method for selecting a vector for expressing a purified and immobilized modified protein of interest, said protein of interest having, after purification and immobilization, at least the same biological activity as the native protein of interest and being directly usable, said method being **characterized in that** it comprises the following steps:

at least four vectors each comprising at least a gene encoding said modified protein of interest, a "polyK" nucleotide fragment encoding a series of at least six lysine residues and a "polyH" nucleotide fragment encoding a series of at least six histidine residues, are provided, the four vectors being chosen from:

(i) a vector comprising from 5' end to 3' end, the polyH nucleotide fragment, cloning sites for the insertion of said gene of interest and on the 3' end the polyK nucleotide fragment;
(ii) a vector comprising from 5' end to 3' end, the polyK nucleotide fragment, cloning sites for the insertion of said gene of interest and on the 3' end the polyH nucleotide fragment;
(iii) a vector comprising from 5' end to 3' end, cloning sites for the insertion of said gene of interest, the polyK nucleotide fragment and the polyH nucleotide fragment;
(iv) a vector comprising from 5' end to 3' end, the polyH nucleotide fragment, the polyK nucleotide fragment and cloning sites for the insertion of said gene of interest;

the nucleotide sequences are expressed in a suitable expression system;
the modified proteins are purified;
the purified modified proteins are immobilized;
the biological activity of the immobilized purified modified proteins is tested; and
the vector expressing the protein that exhibits the highest expression yield and/or the highest purification yield and/or the highest immobilization yield and/or at least the same biological activity as the native protein of interest, is selected.

2. The method as claimed in claim 1, **characterized in that** the polyK fragment encodes a series of six lysine residues, and the polyH fragment encodes a series of six histidine residues.

3. The method as claimed in claim 1 or 2, **characterized in that** the polyH and the polyK nucleotide fragments of the vector (iii) are contiguous.

4. The method as claimed in any one of claims 1 to 3, **characterized in that** the polyH and the polyK nucleotide fragments of the vector (iv) are contiguous.

5. The method as claimed in any one of claims 1, 2 and 4, **characterized in that** a nucleotide sequence encoding a spacer arm is intercalated between the polyH and the polyK nucleotide fragments of the vector (iii), said sequence being selected from SEQ ID NO : 5 to 8.

6. The method as claimed in any one of claims 1, 2 and 3, **characterized in that** a nucleotide sequence encoding a spacer arm is intercalated between the polyH and the polyK nucleotide fragments of the vector (iv), said sequence being selected from SEQ ID NO : 5 to 8.

7. The method as claimed in any one of claims 1 to 6, **characterized in that** the protein of interest is the HIV-1 p24 protein.

8. The method as claimed in claim 7, **characterized in that** the protein of interest is identified by SEQ ID NO: 13 and the modified protein has a sequence chosen from SEQ ID NO: 14 to 20.

9. The method as claimed in any one of claims 1 to 8, **characterized in that** the modified protein is purified by metal ion affinity chromatography.

10. The method as claimed in any one of claims 1 to 9, **characterized in that** the modified protein is immobilized on a linear or particulate polymer.

11. A kit for expressing a purified and immobilized modified protein of interest, said protein of interest having, after purification and immobilization, at least the same biological activity as the native protein of interest and being directly usable, said kit comprising four vectors, each comprising at least a nucleotide sequence which comprises at least a gene encoding said protein of interest, a "polyK" nucleotide fragment encoding a series of at least six lysine residues and a "polyH" nucleotide fragment encoding a series of at least six histidine residues, said kit consisting of the following vectors:

(i) a vector comprising from 5' end to 3' end, the polyH nucleotide fragment, cloning sites for the insertion of said gene of interest and on the 3' end the polyK nucleotide fragment;
(ii) a vector comprising from 5' end to 3' end, the polyK nucleotide fragment, cloning sites for the insertion of said gene of interest and on the 3' end the polyH nucleotide fragment;
(iii) a vector comprising from 5' end to 3' end, cloning sites for the insertion of said gene of interest, the polyK nucleotide fragment and the polyH nucleotide fragment; and
(iv) a vector comprising from 5' end to 3' end, the polyH nucleotide fragment, the polyK nucleotide fragment and cloning sites for the insertion of said gene of interest.

12. The kit as claimed in claim 11, **characterized in that** the polyK fragment encodes a series of six lysine residues, and the polyH fragment encodes a series of six histidine residues.

13. The kit as claimed in claim 11 or 12, **characterized in that** the polyH and the polyK nucleotide fragments of the vector (iii) are contiguous.

**14.** The kit as claimed in any one of claims 11 to 13, **characterized in that** the polyH and the polyK nucleotide fragments of the vector (iv) are contiguous.

**15.** The kit as claimed in any one of claims 11, 12 and 14, **characterized in that** a nucleotide sequence encoding a spacer arm is intercalated between the polyH and the polyK nucleotide fragments of the vector (iii), said sequence being selected from SEQ ID NO : 5 to 8.

**16.** The kit as claimed in any one of claims 11, 12 and 13, **characterized in that** a nucleotide sequence encoding a spacer arm is intercalated between the polyH and the polyK nucleotide fragments of the vector (iv), said sequence being selected from SEQ ID NO : 5 to 8.

**17.** The kit as claimed in any one of claims 11 to 16, **characterized in that** the protein of interest is the HIV-1 p24 protein.

**18.** The method as claimed in claim 17, **characterized in that** the protein is identified by SEQ ID NO: 13 and the modified protein has a sequence chosen from SEQ ID NO: 14 to 20.

**Patentansprüche**

**1.** Verfahren zur Auswahl eines Vektors für die Expression eines modifizierten, gereinigten und immobilisierten Proteins von Interesse, wobei das modifizierte Protein von Interesse nach Reinigung und Immobilisation mindestens dieselbe biologische Aktivität hat wie das native Protein von Interesse und direkt verwendbar ist, wobei das Verfahren **dadurch gekennzeichnet, dass** man in den folgenden Schritten:

über mindestens vier Vektoren verfügt, die jeweils mindestens ein Gen, das für das Protein von Interesse kodiert, ein Nukleotidfragment (polyK), das für eine Aufeinanderfolge von mindestens sechs Lysinresten kodiert, und ein Nukleotidfragment (polyH), das für eine Aufeinanderfolge von mindestens sechs Histidinresten kodiert, umfassen, wobei die Vektoren ausgewählt sind aus:

i) einem Vektor, der von 5' nach 3' das polyH-Nukleotidfragment, Klonierungsstellen zur Insertion des Gens von Interesse und an 3' das polyK-Nukleotidfragment umfasst,
ii) einem Vektor, der von 5' nach 3' das polyK-Nukleotidfragment, Klonierungsstellen zur Insertion des Gens von Interesse und an 3' das polyH-Nukleotidfragment umfasst,
iii) einem Vektor, der von 5' nach 3' Klonierungsstellen zur Insertion des Gens von Interesse, das polyK-Nukleotidfragment und das polyH-Nukleotidfragment umfasst, und
iv) einem Vektor, der von 5' nach 3' das polyH-Nukleotidfragment und das polyK-Nukleotidfragment und Klonierungsstellen zur Insertion des Gens von Interesse, umfasst,

dass man die Nukleotidsequenzen in einem geeigneten Expressionssystem exprimiert,
die modifizierten Proteine reinigt,
die gereinigten modifizierten Proteine immobilisiert,
die biologische Aktivität der modifizierten, gereinigten und immobilisierten Proteine testet und
unter den vorstehenden Vektoren den Vektor, der das Protein exprimiert, auswählt, bei dem die Expression am höchsten ist und/oder bei dem die Reinigungsausbeute am höchsten ist und/oder bei dem die Immobilisationsausbeute am höchsten ist und/oder bei dem die biologische Aktivität zumindest diejenige des nativen Proteins von Interesse ist.

**2.** Verfahren nach Anspruch 1, **dadurch gekennzeichnet, dass** das polyK-Nukleotidfragment für eine Aufeinanderfolge von sechs Lysinresten kodiert und das polyH-Nukleotidfragment für eine Aufeinanderfolge von sechs Histidinresten kodiert.

**3.** Verfahren nach Anspruch 1 oder 2, **dadurch gekennzeichnet, dass** die polyH- und polyK-Nukleotidfragmente des unter iii) genannten Vektors aneinander grenzen.

**4.** Verfahren nach einem der Ansprüche 1 bis 3, **dadurch gekennzeichnet, dass** die polyH- und polyK-Nukleotidfragmente des unter iv) genannten Vektors aneinander grenzen.

**5.** Verfahren nach einem der Ansprüche 1, 2 und 4, **dadurch gekennzeichnet, dass** die polyH- und polyK-Nukleotidfragmente des unter iii) genannten Vektors durch eine Nukleotidsequenz getrennt sind, die für einen Spacerarm kodiert, der aus SEQ ID NR: 5 bis 8 ausgewählt ist.

6. Verfahren nach einem der Ansprüche 1, 2 und 3, **dadurch gekennzeichnet, dass** die polyH- und polyK-Nukleotidfragmente des unter iv) genannten Vektors durch eine Nukleotidsequenz getrennt sind, die für einen Spacerarm kodiert, der aus SEQ ID NR: 5 bis 8 ausgewählt ist.

7. Verfahren nach einem der Ansprüche 1 bis 6, **dadurch gekennzeichnet, dass** das Protein von Interesse das HIV-1-p24-Protein ist.

8. Verfahren nach Anspruch 7, **dadurch gekennzeichnet, dass** das p24-Protein durch SEQ ID NR: 13 identifiziert wird und das modifizierte Protein eine Sequenz aufweist, die aus SEQ ID NR: 14 bis 20 ausgewählt ist.

9. Verfahren nach einem der Ansprüche 1 bis 8, **dadurch gekennzeichnet, dass** man das modifizierte Protein durch Metallionen-Affinitätschromatographie reinigt.

10. Verfahren nach einem der Ansprüche 1 bis 9, **dadurch gekennzeichnet, dass** man das modifizierte Protein auf einem linearen oder teilchenförmigen Polymer immobilisiert.

11. Kit für die Expression eines modifizierten, gereinigten und immobilisierten Proteins von Interesse, wobei das modifizierte Protein von Interesse nach Reinigung und Immobilisation mindestens dieselbe biologische Aktivität wie das native Protein von Interesse besitzt und direkt einsetzbar ist, wobei das Kit vier Vektoren aufweist, die jeweils eine Nukleotidsequenz, die mindestens ein Gen umfasst, das für das Protein von Interesse kodiert, ein Nukleotidfragment (polyK), das für eine Aufeinanderfolge von mindestens sechs Lysinresten kodiert, und ein Nukleotidfragment (polyH), das für eine Aufeinanderfolge von mindestens sechs Histidinresten kodiert, enthalten, wobei das Kit aus den folgenden Vektoren besteht:

i) einem Vektor, der von 5' nach 3' das polyH-Nukleotidfragment, Klonierungsstellen zur Insertion des Gens von Interesse und an 3' das polyK-Nukleotidfragment umfasst,
ii) einem Vektor, der von 5' nach 3' das polyK-Nukleotidfragment, Klonierungsstellen zur Insertion des Gens von Interesse und an 3' das polyH-Nukleotidfragment umfasst,
iii) einem Vektor, der von 5' nach 3' Klonierungsstellen zur Insertion des Gens von Interesse, das polyK-Nukleotidfragment und das polyH-Nukleotidfragment umfasst, und
iv) einem Vektor, der von 5' nach 3' das polyH-Nukleotidfragment und das polyK-Nukleotidfragment und Klonierungsstellen zur Insertion des Gens von Interesse umfasst.

12. Kit nach Anspruch 11, **dadurch gekennzeichnet, dass** das polyK-Nukleotidfragment für eine Aufeinanderfolge von sechs Lysinresten kodiert und das polyH-Nukleotidfragment für eine Aufeinanderfolge von sechs Histidinresten kodiert.

13. Kit nach Anspruch 11 oder 12, **dadurch gekennzeichnet, dass** die polyH- und polyK-Nukleotidfragmente des unter iii) genannten Vektors aneinander grenzen.

14. Kit nach einem der Ansprüche 11 bis 13, **dadurch gekennzeichnet, dass** die polyH- und polyK-Nukleotidfragmente des unter iv) genannten Vektors aneinander grenzen.

15. Kit nach einem der Ansprüche 11, 12 und 14, **dadurch gekennzeichnet, dass** die polyH- und polyK-Nukleotidfragmente des unter iii) genannten Vektors durch eine Nukleotidsequenz getrennt sind, die für einen Spacerarm kodiert, der aus SEQ ID NR: 5 bis 8 ausgewählt ist.

16. Kit nach einem der Ansprüche 11, 12 und 13, **dadurch gekennzeichnet, dass** die polyH- und polyK-Nukleotidfragmente des unter iv) genannten Vektors durch eine Nukleotidsequenz getrennt sind, die für einen Spacerarm kodiert, der aus SEQ ID NR: 5 bis 8 ausgewählt ist.

17. Kit nach einem der Ansprüche 11 bis 16, **dadurch gekennzeichnet, dass** das Protein von Interesse das HIV-1-p24-Protein ist.

18. Kit nach Anspruch 17, **dadurch gekennzeichnet, dass** das p24-Protein durch SEQ ID NR: 13 identifiziert wird und

das modifizierte Protein eine Sequenz aufweist, die aus SEQ ID NR: 14 bis 20 ausgewählt ist.

# FIGURE 1

**A**

**Séquence native de la p24 de l'isolat HXB2**

```
PIVQNIQGQMVHQAISPRTLNAWVKVVEEKAFSPEVIPMFSALSEGATPQ      50
DLNTMLNTVGGHQAAMQMLKETINEEAAEWDRVHPVHAGPIAPGQMREPR     100
GSDIAGTTSTLQEQIGWMTNNPPIPVGEIYKRWIILGLNKIVRMYSPTSI     150
LDIRQGPKEPFRDYVDRFYKTLRAEQASQEVKNWMTETLLVQNANPDCKT     200
ILKALGPAATLEEMMTACQGVGGPGHKARVL                        231
```

**B**

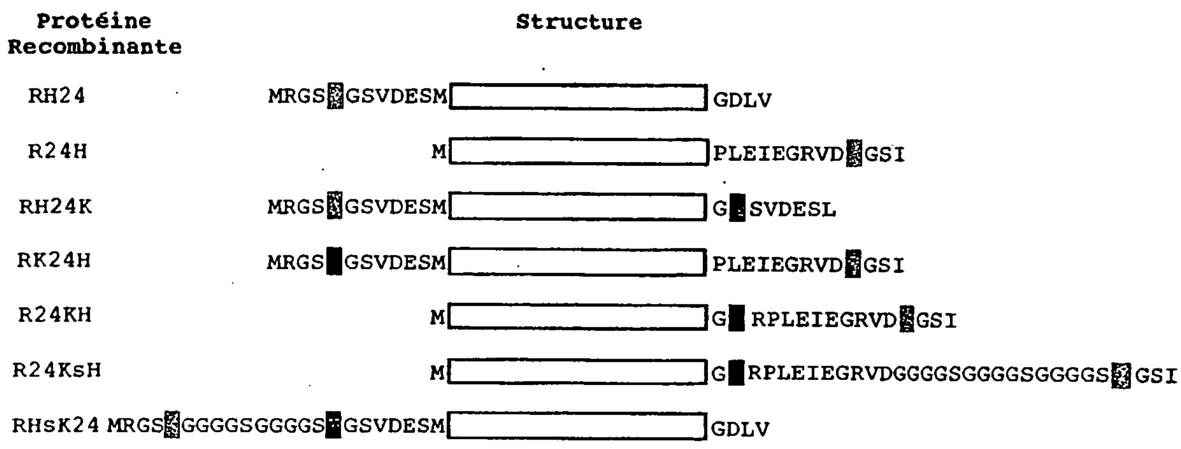

FIGURE 2

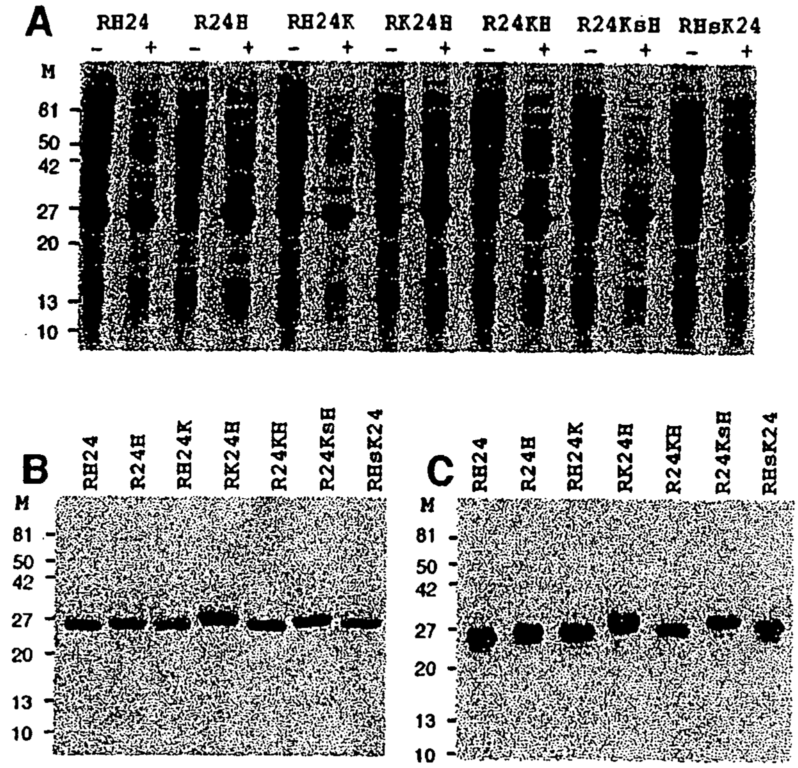


FIGURE 3

| | Acides Aminés | MM théorique a | MM déterminée par LC/ESI/Ms[a] |
|---|---|---|---|
| RH24 | 243 | 26950 | 26961 |
| R24H | 241 | 26826 | 26703 |
| RH24K | 252 | 28022 | 28032 |
| RK24H | 257 | 28601 | 28612 |
| R24KH | 249 | 27808 | 27687 |
| R24KsH | 264 | 28754 | 28635 |
| RHsK24 | 259 | 28349 | 28372 |

## FIGURE 4

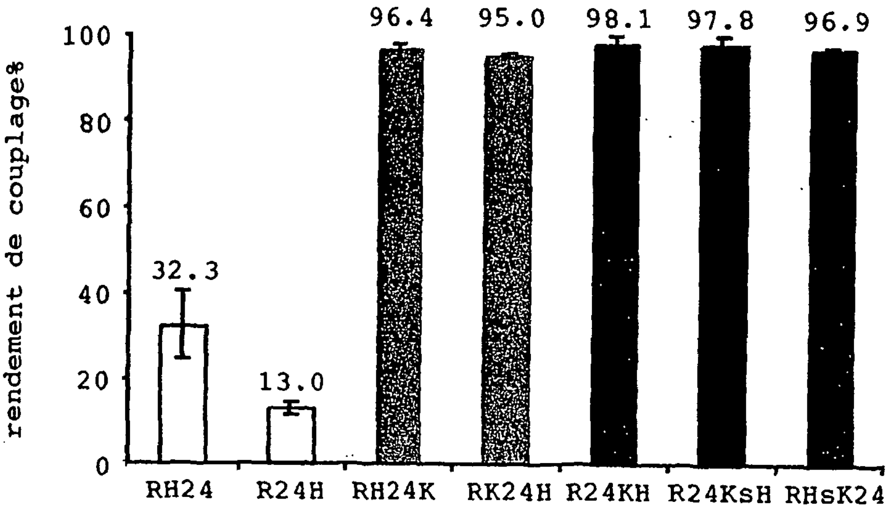


## FIGURE 5

| Echantillon testé[a] | N-Terminal[b] | | C-Terminal[b] | | | |
|---|---|---|---|---|---|---|
| | 15F8 (250 ng/ml)[c] | | 23A5 (0,75 ng/ml)[c] | | 3D8 (0,06 ng/ml)[c] | |
| | DO492 nm | $DO_{ET}/DO_{Ref}$[d] | DO492 nm | $DO_{ET}/DO_{Ref}$[d] | DO492 nm | $DO_{ET}/DO_{Ref}$[d] |
| RH24K-AMVE67 | 1507 | 1 | 248 | 1 | 665 | 1 |
| RK24H-AMVE67 | 1088 | 0,7 | 2054 | 8,3 | 1498 | 2,25 |

## FIGURE 6

**A**

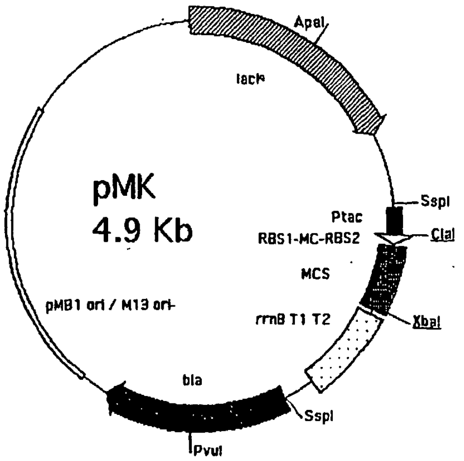


**B**

**pMK81 vector**

```
                             ClaI
CACAGGAAACAGAATTAATAatgtatcgattaaataaggaggaataaCATATGAGGGGAT
   RBS1                                     RBS2         M  R  G
                                         NcoI      EcoRI      SacI
CCCACCATCACCATCACCACGGTTCTGTCGACGAATCCATGGACGAATTCGAGCTCGGTA
S  H  H  H  H  H  H  G  S  V  D  E  S  M  .  .  .  .  .  .  .
KpnI BglII XhoI    PstI  SphI HindIII SmaI
CCCGGAGATCTCTCGAGCTGCAGCATGCAAGCTTCCCGGGAAGAAGAAGAAGAAGAAGTC
 .  .  .  .  .  .  .  .  .  .  .  .  G  K  K  K  K  K  K  S
                       XbaI
  TGTCGACGAATCTCTCTAGTCTAGA
    V  D  E  S  L
```

**pMK82 vector**

```
                             ClaI                     NcoI    EcoRI
CACAGGAAACAGAATTAATAatgtatcgattaaataaggaggaataaACCATGGACGAAT
   RBS1                                     RBS2       M  D  E
      SacI KpnI BglII                PstI SphI HindIII SmaI
TCGAGCTCGGTACCCGGAGATCTCTCGAGCTGCAGCATGCAAGCTTCCCGGGAAGAAGAA
 .  .  .  .  .  .  .  .  .  .  .  .  .  .  .  .  G  K  K  K

GAAGAAGAAGAGGCCTCTCGAGATCGAAGGTCGGGTCGACCACCATCACCATCACCACGG
  K  K  K  R  P  L  E  I  E  G  R  V  D  H  H  H  H  H  H  G
     XbaI
ATCCATCTAGA
  S  I
```

**pMK83 vector**

```
                             ClaI
CACAGGAAACAGAATTAATAatgtatcgattaaataaggaggaataaCATATGAGGGGAT
   RBS1                                     RBS2         M  R  G
                                         NcoI      EcoRI      SacI
CCAAGAAGAAGAAGAAGAAGGGTTCTGTCGACGAATCCATGGACGAATTCGAGCTCGGTA
S  K  K  K  K  K  K  G  S  V  D  E  S  M  .  .  .  .  .  .
KpnI BglII XhoI    PstI  SphI HindIII SmaI
CCCGGAGATCTCTCGAGCTGCAGCATGCAAGCTTCCCGGGATCGAGATCGAAGGTCGGGT
 .  .  .  .  .  .  .  .  .  .  .  .  G  I  E  I  E  G  R  V
                                  XbaI
CGACCACCATCACCATCACCACGGATCCATCTAGA
  D  H  H  H  H  H  H  G  S  I
```

**pMK84 vector**

```
                             ClaI
CACAGGAAACAGAATTAATAatgtatcgattaaataaggaggaataaCATATGAGGGGAT
   RBS1                                     RBS2         M  R  G

CCCACCATCACCATCACCACGGTGGAGGTGGATCTGGTGGAGGTGGATCTAAGAAGAAGA
S  H  H  H  H  H  H  G  G  G  G  S  G  G  G  G  S  K  K  K
                             NcoI    EcoRI     SacI  KpnI BglII
AGAAGAAGGGTTCTGTCGACGAATCCATGGACGAATTCGAGCTCGGTACCCGGAGATCTC
K  K  K  G  S  V  D  E  S  M  .  .  .  .  .  .  .  .  .  .
XhoI     PstI  SphI HindIII SmaI        XbaI
 TCGAGCTGCAGCATGCAAGCTTCCCGGGGATCTAGTTAGA
   .  .  .  .  .  .  .  .  G  D  L  V  .
```

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9859241 A
- US 5916794 A
- WO 9845449 A, Arnaud
- FR 0115081


### Littérature non-brevet citée dans la description


- **E. Hoculi et al.** Bio/Technology. Nature Publishing Co, Novembre 1988, 1321-1325
- **Monfardini C. ; F. M. Veronese.** *Stabilization of Substances in Circulation (review) Bioconjugate Chem.,* 1998, vol. 9, 418-450
- **Duncan R.** Polymer conjugates for tumour targeting and intracytoplasmic delivery. The EPR effect as a common gateway?. *Pharmaceutical Science & Technology Today,* 1999, vol. 2 (11), 441-449
- **Varga C.M. ; Wickham T.J. ; D.A. Lauffenburger.** Receptor-mediated targeting of gene delivery vectors: Insights from molecular mechanisms for improved vehicle design (Review). *Biotechnology and Bioengineering,* 2000, vol. 70 (6), 593-605
- **Ladavière C. ; T. Delair ; A. Domard ; A. Novelli-Rousseau ; B. Mandrand ; F. Mallet.** Covalent immobilization of proteins onto (maleic anhydride-a/t-methyl vinyl ether) copolymers: enhanced immobilization of recombinant proteins. *Bioconjug Chem,* 1998, vol. 9 (6), 655-661
- **Laure Allard ; Valérie Cheynet ; Guy Oriol ; Laurent Véron ; Françoise Merlier ; Gérald Scrémin ; Bernard Mandrand ; Thierry Delair ; Franois Mallet.** Mechanisms Leading to an Oriented Immobilization of Recombinant Proteins Derived from the p24 Capsid of HIV-1 onto Copolymers. *Bioconjug Chem,* 2001
- **Cheynet, V. ; B. Verrier ; F. Mallet.** Overexpression of HIV-1 proteins in Escherichia coli by a modified expression vector and their one-step purification. *Prot Express Purif,* 1993, vol. 4, 367-372
- **Berthet-Colominas C. ; S. Monaco ; A.Novelli ; G. Sibaï ; F. Mallet ; S. Cusack.** Head-to-tail dimers and interdomain flexibility revealed by the crystal structure of HIV-1 capsid protein (p24) complexed with a monoclonal antibody. *Fab. EMBO,* 1999, vol. 18 (5), 1124-1136
- **Arnaud N. ; V. Cheynet ; G. Oriol ; B. Mandrand ; F. Mallet.** Construction and expression of a modular gene encoding bacteriophage T7 RNA polymerase. *Gene,* 1997, vol. 199 (1-2), 19-156
- **Ganachaud F. ; Mouterde G ; Delair T ; Elaïssari A. ; Pichot C.** Preparation and characterization of cationic polystyrene latex particles of different aminated surface charges. *Polymers for Advanced Technologies,* 1995, vol. 6, 480-488

LISTE DE SEQUENCES

<110> bioMérieux

<120> SEQUENCE NUCLEOTIDIQUE CODANT POUR UNE PROTEINE D'INTERET MODIFIEE, VECTEUR D'EXPRESSION ET PROCEDE D'OBTENTION

<130> pMK

<140>
<141>

<150> FR0115081
<151> 2001-11-21

<160> 20

<170> PatentIn Ver. 2.1

<210> 1
<211> 4935
<212> ADN
<213> Séquence artificielle

<220>
<223> Description de la séquence artificielle: plasmide pMK81

<400> 1

```
ccgacaccat cgaatggcgc aaaacctttc gcggtatggc atgatagcgc ccggaagaga 60
gtcaattcag ggtggtgaat gtgaaaccag taacgttata cgatgtcgca gagtatgccg 120
gtgtctctta tcagaccgtt tcccgcgtgg tgaaccaggc cagccacgtt tctgcgaaaa 180
cgcgggaaaa agtggaagcg gcgatggcgg agctgaatta cattcccaac cgcgtggcac 240
aacaactggc gggcaaacag tcgttgctga ttggcgttgc cacctccagt ctggccctgc 300
acgcgccgtc gcaaattgtc gcggcgatta aatctcgcgc cgatcaactg ggtgccagcg 360
tggtggtgtc gatggtagaa cgaagcggcg tcgaagcctg taaagcggcg gtgcacaatc 420
ttctcgcgca acgcgtcagt gggctgatca ttaactatcc gctggatgac caggatgcca 480
ttgctgtgga agctgcctgc actaatgttc cggcgttatt tcttgatgtc tctgaccaga 540
cacccatcaa cagtattatt ttctcccatg aagacggtac gcgactgggc gtggagcatc 600
tggtcgcatt gggtcaccag caaatcgcgc tgttagcggg cccattaagt tctgtctcgg 660
cgcgtctgcg tctggctggc tggcataaat atctcactcg caatcaaatt cagccgatag 720
cggaacggga aggcgactgg agtgccatgt ccggttttca acaaaccatg caaatgctga 780
atgagggcat cgttcccact gcgatgctgg ttgccaacga tcagatggcg ctgggcgcaa 840
tgcgcgccat taccgagtcc gggctgcgcg ttggtgcgga tatctcggta gtgggatacg 900
acgataccga agacagctca tgttatatcc cgccgttaac caccatcaaa caggattttc 960
gcctgctggg gcaaaccagc gtggaccgct tgctgcaact ctctcagggc caggcggtga 1020
agggcaatca gctgttgccc gtctcactgg tgaaaagaaa aaccaccctg gcgcccaata 1080
```

cgcaaaccgc ctctccccgc gcgttggccg attcattaat gcagctggca cgacaggttt 1140
cccgactgga aagcgggcag tgagcgcaac gcaattaatg tgagttagct cactcattag 1200
gcacaattct catgtttgac agcttatcat cgactgcacg gtgcaccaat gcttctggcg 1260
tcaggcagcc atcggaagct gtggtatggc tgtgcaggtc gtaaatcact gcataattcg 1320
tgtcgctcaa ggcgcactcc cgttctggat aatgtttttt gcgccgacat cataacggtt 1380
ctggcaaata tttctgaaat gagctgttga caattaatca tcggctcgta taatgtgtgg 1440
aattgtgagc ggataacaat ttcacacagg aaacagaatt aataatgtat cgattaaata 1500
aggaggaata acatatgagg ggatcccacc atcaccatca ccacggttct gtcgacgaat 1560
ccatggacga attcgagctc ggtacccgga gatctctcga gctgcagcat gcaagcttcc 1620
cgggaagaag aagaagaaga agtctgtcga cgaatctctc tagtctagac tagagcttag 1680
cttggctgtt ttggcggatg agagaagatt ttcagcctga tacagattaa atcagaacgc 1740
agaagcggtc tgataaaaca gaatttgcct ggcggcagta gcgcggtggt cccacctgac 1800
cccatgccga actcagaagt gaaacgccgt agcgccgatg gtagtgtggg gtctccccat 1860
gcgagagtag ggaactgcca ggcatcaaat aaaacgaaag gctcagtcga aagactgggc 1920
ctttcgtttt atctgttgtt tgtcggtgaa cgctctcctg agtaggacaa atccgccggg 1980
agcggatttg aacgttgcga agcaacggcc cggagggtgg cgggcaggac gcccgccata 2040
aactgccagg catcaaatta agcagaaggc catcctgacg gatggccttt ttgcgtttct 2100
acaaactctt ttgtttattt ttctaaatac attcaaatat gtatccgctc atgagacaat 2160
aaccctgata aatgcttcaa taatattgaa aaaggaagag tatgagtatt caacatttcc 2220
gtgtcgccct tattcccttt tttgcggcat tttgccttcc tgtttttgct cacccagaaa 2280
cgctggtgaa agtaaaagat gctgaagatc agttgggtgc acgagtgggt tacatcgaac 2340
tggatctcaa cagcggtaag atccttgaga gttttcgccc cgaagaacgt tttccaatga 2400
tgagcacttt taaagttctg ctatgtggcg cggtattatc ccgtgttgac gccgggcaag 2460
agcaactcgg tcgccgcata cactattctc agaatgactt ggttgagtac tcaccagtca 2520
cagaaaagca tcttacggat ggcatgacag taagagaatt atgcagtgct gccataacca 2580
tgagtgataa cactgcggcc aacttacttc tgacaacgat cggaggaccg aaggagctaa 2640
ccgctttttt gcacaacatg ggggatcatg taactcgcct tgatcgttgg gaaccggagc 2700
tgaatgaagc cataccaaac gacgagcgtg acaccacgat gcctgtagca atggcaacaa 2760
cgttgcgcaa actattaact ggcgaactac ttactctagc ttcccggcaa caattaatag 2820
actggatgga ggcggataaa gttgcaggac cacttctgcg ctcggccctt ccggctggct 2880
ggtttattgc tgataaatct ggagccggtg agcgtgggtc tcgcggtatc attgcagcac 2940
tggggccaga tggtaagccc tcccgtatcg tagttatcta cacgacgggg agtcaggcaa 3000
ctatggatga acgaaataga cagatcgctg agataggtgc ctcactgatt aagcattggt 3060
aactgtcaga ccaagtttac tcatatatac tttagattga tttaaaactt catttttaat 3120
ttaaaaggat ctaggtgaag atcctttttg ataatctcat gaccaaaatc ccttaacgtg 3180
agttttcgtt ccactgagcg tcagaccccg tagaaaagat caaaggatct tcttgagatc 3240
cttttttct gcgcgtaatc tgctgcttgc aaacaaaaaa accaccgcta ccagcggtgg 3300
tttgtttgcc ggatcaagag ctaccaactc tttttccgaa ggtaactggc ttcagcagag 3360
cgcagatacc aaatactgtc cttctagtgt agccgtagtt aggccaccac ttcaagaact 3420
ctgtagcacc gcctacatac ctcgctctgc taatcctgtt accagtggct gctgccagtg 3480
gcgataagtc gtgtcttacc gggttggact caagacgata gttaccggat aaggcgcagc 3540
ggtcgggctg aacggggggt tcgtgcacac agcccagctt ggagcgaacg acctacaccg 3600
aactgagata cctacagcgt gagctatgag aaagcgccac gcttcccgaa gggagaaagg 3660
cggacaggta tccggtaagc ggcagggtcg gaacaggaga gcgcacgagg gagcttccag 3720
ggggaaacgc ctggtatctt tatagtcctg tcgggtttcg ccacctctga cttgagcgtc 3780
gatttttgtg atgctcgtca ggggggcgga gcctatggaa aaacgccagc aacgcggcct 3840
ttttacggtt cctggccttt tgctggcctt ttgctcacat gttctttcct gcgttatccc 3900
ctgattctgt ggataaccgt attaccgcct ttgagtgagc tgataccgct cgccgcagcc 3960

```
gaacgaccga gcgcagcgag tcagtgagcg aggaagcgga agagcgcctg atgcggtatt 4020
ttctccttac gcatctgtgc ggtatttcac accgcatatg gtgcactctc agtacaatct 4080
gctctgatgc cgcatagtta agccagtata cactccgcta tcgctacgtg actgggtcat 4140
ggctgcgccc cgacacccgc caacacccgc tgacgcgccc tgacgggctt gtctgctccc 4200
ggcatccgct tacagacaag ctgtgaccgt ctccgggagc tgcatgtgtc agaggttttc 4260
accgtcatca ccgaaacgcg cgaggcagct gcggtaaagc tcatcagcgt ggtcgtgaag 4320
cgattcacag atgtctgcct gttcatccgc gtccagctcg ttgagtttct ccagaagcgt 4380
taatgtctgg cttctgataa agcgggccat gttaagggcg gttttttcct gtttggtcac 4440
ttgatgcctc cgtgtaaggg ggaatttctg ttcatggggg taatgatacc gatgaaacga 4500
gagaggatgc tcacgatacg ggttactgat gatgaacatg cccggttact ggaacgttgt 4560
gagggtaaac aactggcggt atggatgcgg cgggaccaga gaaaaatcac tcagggtcaa 4620
tgccagcgct tcgttaatac agatgtaggt gttccacagg gtagccagca gcatcctgcg 4680
atgcagatcc ggaacataat ggtgcagggc gctgacttcc gcgtttccag actttacgaa 4740
acacggaaac cgaagaccat tcatgttgtt gctcaggtcg cagacgtttt gcagcagcag 4800
tcgcttcacg ttcgctcgcg tatcggtgat tcattctgct aaccagtaag gcaaccccgc 4860
cagcctagcc gggtcctcaa cgacaggagc acgatcatgc gcacccgtgg ccaggaccca 4920
acgctgcccg aaatt                                                  4935
```

<210> 2
<211> 4921
<212> ADN
<213> Séquence artificielle

<220>
<223> Description de la séquence artificielle: plasmide pMK82

<400> 2

```
ccgacaccat cgaatggcgc aaaacctttc gcggtatggc atgatagcgc ccggaagaga 60
gtcaattcag ggtggtgaat gtgaaaccag taacgttata cgatgtcgca gagtatgccg 120
gtgtctctta tcagaccgtt tcccgcgtgg tgaaccaggc cagccacgtt tctgcgaaaa 180
cgcgggaaaa agtggaagcg gcgatggcgg agctgaatta cattcccaac cgcgtggcac 240
aacaactggc gggcaaacag tcgttgctga ttggcgttgc cacctccagt ctggccctgc 300
acgcgccgtc gcaaattgtc gcggcgatta aatctcgcgc cgatcaactg ggtgccagcg 360
tggtggtgtc gatggtagaa cgaagcggcg tcgaagcctg taaagcggcg gtgcacaatc 420
ttctcgcgca acgcgtcagt gggctgatca ttaactatcc gctggatgac caggatgcca 480
ttgctgtgga agctgcctgc actaatgttc cggcgttatt tcttgatgtc tctgaccaga 540
cacccatcaa cagtattatt ttctcccatg aagacggtac gcgactgggc gtggagcatc 600
tggtcgcatt gggtcaccag caaatcgcgc tgttagcggg cccattaagt tctgtctcgg 660
cgcgtctgcg tctggctggc tggcataaat atctcactcg caatcaaatt cagccgatag 720
cggaacggga aggcgactgg agtgccatgt ccggttttca acaaaccatg caaatgctga 780
atgagggcat cgttcccact gcgatgctgg ttgccaacga tcagatggcg ctgggcgcaa 840
tgcgcgccat taccgagtcc gggctgcgcg ttggtgcgga tatctcggta gtgggatacg 900
acgataccga agacagctca tgttatatcc cgccgttaac caccatcaaa caggattttc 960
gcctgctggg gcaaaccagc gtggaccgct tgctgcaact ctctcagggc caggcggtga 1020
agggcaatca gctgttgccc gtctcactgg tgaaaagaaa aaccaccctg gcgcccaata 1080
cgcaaaccgc ctctccccgc gcgttggccg attcattaat gcagctggca cgacaggttt 1140
```

```
cccgactgga aagcgggcag tgagcgcaac gcaattaatg tgagttagct cactcattag 1200
gcacaattct catgtttgac agcttatcat cgactgcacg gtgcaccaat gcttctggcg 1260
tcaggcagcc atcggaagct gtggtatggc tgtgcaggtc gtaaatcact gcataattcg 1320
tgtcgctcaa ggcgcactcc cgttctggat aatgtttttt gcgccgacat cataacggtt 1380
ctggcaaata tttctgaaat gagctgttga caattaatca tcggctcgta taatgtgtgg 1440
aattgtgagc ggataacaat ttcacacagg aaacagaatt aataatgtat cgattaaata 1500
aggaggaata aaccatggac gaattcgagc tcggtacccg gagatctctc gagctgcagc 1560
atgcaagctt cccgggaaga agaagaagaa gaagaggcct ctcgagatcg aaggtcgggt 1620
cgaccaccat caccatcacc acggatccat ctagactaga gcttagcttg gctgttttgg 1680
cggatgagag aagattttca gcctgataca gattaaatca gaacgcagaa gcggtctgat 1740
aaaacagaat ttgcctggcg gcagtagcgc ggtggtccca cctgacccca tgccgaactc 1800
agaagtgaaa cgccgtagcg ccgatggtag tgtggggtct ccccatgcga gagtagggaa 1860
ctgccaggca tcaaataaaa cgaaaggctc agtcgaaaga ctgggccttt cgttttatct 1920
gttgtttgtc ggtgaacgct ctcctgagta ggacaaatcc gccgggagcg gatttgaacg 1980
ttgcgaagca acggcccgga gggtggcggg caggacgccc gccataaact gccaggcatc 2040
aaattaagca gaaggccatc ctgacggatg gcctttttgc gtttctacaa actcttttgt 2100
ttatttttct aaatacattc aaatatgtat ccgctcatga gacaataacc ctgataaatg 2160
cttcaataat attgaaaaag gaagagtatg agtattcaac atttccgtgt cgcccttatt 2220
cccttttttg cggcattttg ccttcctgtt tttgctcacc cagaaacgct ggtgaaagta 2280
aaagatgctg aagatcagtt gggtgcacga gtgggttaca tcgaactgga tctcaacagc 2340
ggtaagatcc ttgagagttt tcgccccgaa gaacgttttc caatgatgag cacttttaaa 2400
gttctgctat gtggcgcggt attatcccgt gttgacgccg ggcaagagca actcggtcgc 2460
cgcatacact attctcagaa tgacttggtt gagtactcac cagtcacaga aaagcatctt 2520
acggatggca tgacagtaag agaattatgc agtgctgcca taaccatgag tgataacact 2580
gcggccaact tacttctgac aacgatcgga ggaccgaagg agctaaccgc ttttttgcac 2640
aacatggggg atcatgtaac tcgccttgat cgttgggaac cggagctgaa tgaagccata 2700
ccaaacgacg agcgtgacac cacgatgcct gtagcaatgg caacaacgtt gcgcaaacta 2760
ttaactggcg aactacttac tctagcttcc cggcaacaat taatagactg gatggaggcg 2820
gataaagttg caggaccact tctgcgctcg gcccttccgg ctggctggtt tattgctgat 2880
aaatctggag ccggtgagcg tgggtctcgc ggtatcattg cagcactggg gccagatggt 2940
aagccctccc gtatcgtagt tatctacacg acggggagtc aggcaactat ggatgaacga 3000
aatagacaga tcgctgagat aggtgcctca ctgattaagc attggtaact gtcagaccaa 3060
gtttactcat atatacttta gattgattta aaacttcatt tttaatttaa aaggatctag 3120
gtgaagatcc tttttgataa tctcatgacc aaaatccctt aacgtgagtt ttcgttccac 3180
tgagcgtcag accccgtaga aaagatcaaa ggatcttctt gagatccttt ttttctgcgc 3240
gtaatctgct gcttgcaaac aaaaaaacca ccgctaccag cggtggtttg tttgccggat 3300
caagagctac caactctttt tccgaaggta actggcttca gcagagcgca gataccaaat 3360
actgtccttc tagtgtagcc gtagttaggc caccacttca agaactctgt agcaccgcct 3420
acatacctcg ctctgctaat cctgttacca gtggctgctg ccagtggcga taagtcgtgt 3480
cttaccgggt tggactcaag acgatagtta ccggataagg cgcagcggtc gggctgaacg 3540
gggggttcgt gcacacagcc cagcttggag cgaacgacct acaccgaact gagataccta 3600
cagcgtgagc tatgagaaag cgccacgctt cccgaaggga gaaaggcgga caggtatccg 3660
gtaagcggca gggtcggaac aggagagcgc acgagggagc ttccaggggg aaacgcctgg 3720
tatctttata gtcctgtcgg gtttcgccac ctctgacttg agcgtcgatt tttgtgatgc 3780
tcgtcagggg ggcggagcct atggaaaaac gccagcaacg cggccttttt acggttcctg 3840
gccttttgct ggccttttgc tcacatgttc tttcctgcgt tatcccctga ttctgtggat 3900
aaccgtatta ccgcctttga gtgagctgat accgctcgcc gcagccgaac gaccgagcgc 3960
agcgagtcag tgagcgagga agcggaagag cgcctgatgc ggtattttct ccttacgcat 4020
```

```
ctgtgcggta tttcacaccg catatggtgc actctcagta caatctgctc tgatgccgca 4080
tagttaagcc agtatacact ccgctatcgc tacgtgactg ggtcatggct gcgccccgac 4140
acccgccaac acccgctgac gcgccctgac gggcttgtct gctcccggca tccgcttaca 4200
gacaagctgt gaccgtctcc gggagctgca tgtgtcagag gttttcaccg tcatcaccga 4260
aacgcgcgag gcagctgcgg taaagctcat cagcgtggtc gtgaagcgat tcacagatgt 4320
ctgcctgttc atccgcgtcc agctcgttga gtttctccag aagcgttaat gtctggcttc 4380
tgataaagcg ggccatgtta agggcggttt tttcctgttt ggtcacttga tgcctccgtg 4440
taagggggaa tttctgttca tgggggtaat gataccgatg aaacgagaga ggatgctcac 4500
gatacgggtt actgatgatg aacatgcccg gttactggaa cgttgtgagg gtaaacaact 4560
ggcggtatgg atgcggcggg accagagaaa aatcactcag ggtcaatgcc agcgcttcgt 4620
taatacagat gtaggtgttc cacagggtag ccagcagcat cctgcgatgc agatccggaa 4680
cataatggtg cagggcgctg acttccgcgt ttccagactt tacgaaacac ggaaaccgaa 4740
gaccattcat gttgttgctc aggtcgcaga cgttttgcag cagcagtcgc ttcacgttcg 4800
ctcgcgtatc ggtgattcat tctgctaacc agtaaggcaa ccccgccagc ctagccgggt 4860
cctcaacgac aggagcacga tcatgcgcac ccgtggccag gacccaacgc tgcccgaaat 4920
t                                                                  4921
```

<210> 3
<211> 4945
<212> ADN
<213> Séquence artificielle

<220>
<223> Description de la séquence artificielle: plasmide pMK83

<400> 3

```
ccgacaccat cgaatggcgc aaaacctttc gcggtatggc atgatagcgc ccggaagaga 60
gtcaattcag ggtggtgaat gtgaaaccag taacgttata cgatgtcgca gagtatgccg 120
gtgtctctta tcagaccgtt tcccgcgtgg tgaaccaggc cagccacgtt tctgcgaaaa 180
cgcgggaaaa agtggaagcg gcgatggcgg agctgaatta cattcccaac cgcgtggcac 240
aacaactggc gggcaaacag tcgttgctga ttggcgttgc cacctccagt ctggccctgc 300
acgcgccgtc gcaaattgtc gcggcgatta aatctcgcgc cgatcaactg ggtgccagcg 360
tggtggtgtc gatggtagaa cgaagcggcg tcgaagcctg taaagcggcg gtgcacaatc 420
ttctcgcgca acgcgtcagt gggctgatca ttaactatcc gctggatgac caggatgcca 480
ttgctgtgga agctgcctgc actaatgttc cggcgttatt tcttgatgtc tctgaccaga 540
cacccatcaa cagtattatt ttctcccatg aagacggtac gcgactgggc gtggagcatc 600
tggtcgcatt gggtcaccag caaatcgcgc tgttagcggg cccattaagt tctgtctcgg 660
cgcgtctgcg tctggctggc tggcataaat atctcactcg caatcaaatt cagccgatag 720
cggaacggga aggcgactgg agtgccatgt ccggttttca acaaaccatg caaatgctga 780
atgagggcat cgttcccact gcgatgctgg ttgccaacga tcagatggcg ctgggcgcaa 840
tgcgcgccat taccgagtcc gggctgcgcg ttggtgcgga tatctcggta gtgggatacg 900
acgataccga agacagctca tgttatatcc cgccgttaac caccatcaaa caggattttc 960
gcctgctggg gcaaaccagc gtggaccgct tgctgcaact ctctcagggc caggcggtga 1020
agggcaatca gctgttgccc gtctcactgg tgaaaagaaa aaccaccctg gcgcccaata 1080
cgcaaaccgc ctctccccgc gcgttggccg attcattaat gcagctggca cgacaggttt 1140
cccgactgga aagcgggcag tgagcgcaac gcaattaatg tgagttagct cactcattag 1200
```

```
gcacaattct catgtttgac agcttatcat cgactgcacg gtgcaccaat gcttctggcg 1260
tcaggcagcc atcggaagct gtggtatggc tgtgcaggtc gtaaatcact gcataattcg 1320
tgtcgctcaa ggcgcactcc cgttctggat aatgtttttt gcgccgacat cataacggtt 1380
ctggcaaata tttctgaaat gagctgttga caattaatca tcggctcgta taatgtgtgg 1440
aattgtgagc ggataacaat ttcacacagg aaacagaatt aataatgtat cgattaaata 1500
aggaggaata acatatgagg ggatccaaga agaagaagaa gaagggttct gtcgacgaat 1560
ccatggacga attcgagctc ggtacccgga gatctctcga gctgcagcat gcaagcttcc 1620
cgggatcgag atcgaaggtc gggtcgacca ccatcaccat caccacggat ccatctagac 1680
tagagcttag cttggctgtt ttggcggatg agagaagatt ttcagcctga tacagattaa 1740
atcagaacgc agaagcggtc tgataaaaca gaatttgcct ggcggcagta gcgcggtggt 1800
cccacctgac cccatgccga actcagaagt gaaacgccgt agcgccgatg gtagtgtggg 1860
gtctccccat gcgagagtag ggaactgcca ggcatcaaat aaaacgaaag gctcagtcga 1920
aagactgggc ctttcgtttt atctgttgtt tgtcggtgaa cgctctcctg agtaggacaa 1980
atccgccggg agcggatttg aacgttgcga agcaacggcc cggagggtgg cgggcaggac 2040
gcccgccata aactgccagg catcaaatta agcagaaggc catcctgacg gatggccttt 2100
ttgcgtttct acaaactctt ttgtttattt ttctaaatac attcaaatat gtatccgctc 2160
atgagacaat aaccctgata aatgcttcaa taatattgaa aaaggaagag tatgagtatt 2220
caacatttcc gtgtcgccct tattcccttt tttgcggcat tttgccttcc tgtttttgct 2280
cacccagaaa cgctggtgaa agtaaaagat gctgaagatc agttgggtgc acgagtgggt 2340
tacatcgaac tggatctcaa cagcggtaag atccttgaga gttttcgccc cgaagaacgt 2400
tttccaatga tgagcacttt taaagttctg ctatgtggcg cggtattatc ccgtgttgac 2460
gccgggcaag agcaactcgg tcgccgcata cactattctc agaatgactt ggttgagtac 2520
tcaccagtca cagaaaagca tcttacggat ggcatgacag taagagaatt atgcagtgct 2580
gccataacca tgagtgataa cactgcggcc aacttacttc tgacaacgat cggaggaccg 2640
aaggagctaa ccgctttttt gcacaacatg ggggatcatg taactcgcct tgatcgttgg 2700
gaaccggagc tgaatgaagc cataccaaac gacgagcgtg acaccacgat gcctgtagca 2760
atggcaacaa cgttgcgcaa actattaact ggcgaactac ttactctagc ttcccggcaa 2820
caattaatag actggatgga ggcggataaa gttgcaggac cacttctgcg ctcggccctt 2880
ccggctggct ggtttattgc tgataaatct ggagccggtg agcgtgggtc tcgcggtatc 2940
attgcagcac tggggccaga tggtaagccc tcccgtatcg tagttatcta cacgacgggg 3000
agtcaggcaa ctatggatga acgaaataga cagatcgctg agataggtgc ctcactgatt 3060
aagcattggt aactgtcaga ccaagtttac tcatatatac tttagattga tttaaaactt 3120
catttttaat ttaaaaggat ctaggtgaag atcctttttg ataatctcat gaccaaaatc 3180
ccttaacgtg agttttcgtt ccactgagcg tcagaccccg tagaaaagat caaaggatct 3240
tcttgagatc ctttttttct gcgcgtaatc tgctgcttgc aaacaaaaaa accaccgcta 3300
ccagcggtgg tttgtttgcc ggatcaagag ctaccaactc tttttccgaa ggtaactggc 3360
ttcagcagag cgcagatacc aaatactgtc cttctagtgt agccgtagtt aggccaccac 3420
ttcaagaact ctgtagcacc gcctacatac ctcgctctgc taatcctgtt accagtggct 3480
gctgccagtg gcgataagtc gtgtcttacc gggttggact caagacgata gttaccggat 3540
aaggcgcagc ggtcgggctg aacggggggt tcgtgcacac agcccagctt ggagcgaacg 3600
acctacaccg aactgagata cctacagcgt gagctatgag aaagcgccac gcttcccgaa 3660
gggagaaagg cggacaggta tccggtaagc ggcagggtcg gaacaggaga gcgcacgagg 3720
gagcttccag ggggaaacgc ctggtatctt tatagtcctg tcgggtttcg ccacctctga 3780
cttgagcgtc gatttttgtg atgctcgtca ggggggcgga gcctatggaa aaacgccagc 3840
aacgcggcct ttttacggtt cctggccttt tgctggcctt ttgctcacat gttctttcct 3900
gcgttatccc ctgattctgt ggataaccgt attaccgcct ttgagtgagc tgataccgct 3960
cgccgcagcc gaacgaccga gcgcagcgag tcagtgagcg aggaagcgga agagcgcctg 4020
atgcggtatt ttctccttac gcatctgtgc ggtatttcac accgcatatg gtgcactctc 4080
```

```
agtacaatct gctctgatgc cgcatagtta agccagtata cactccgcta tcgctacgtg 4140
actgggtcat ggctgcgccc cgacacccgc caacacccgc tgacgcgccc tgacgggctt 4200
gtctgctccc ggcatccgct tacagacaag ctgtgaccgt ctccgggagc tgcatgtgtc 4260
agaggttttc accgtcatca ccgaaacgcg cgaggcagct gcggtaaagc tcatcagcgt 4320
ggtcgtgaag cgattcacag atgtctgcct gttcatccgc gtccagctcg ttgagtttct 4380
ccagaagcgt taatgtctgg cttctgataa agcgggccat gttaagggcg gtttttttcct 4440
gtttggtcac ttgatgcctc cgtgtaaggg ggaatttctg ttcatggggg taatgatacc 4500
gatgaaacga gagaggatgc tcacgatacg ggttactgat gatgaacatg cccggttact 4560
ggaacgttgt gagggtaaac aactggcggt atggatgcgg cgggaccaga gaaaaatcac 4620
tcagggtcaa tgccagcgct tcgttaatac agatgtaggt gttccacagg gtagccagca 4680
gcatcctgcg atgcagatcc ggaacataat ggtgcagggc gctgacttcc gcgtttccag 4740
actttacgaa acacggaaac cgaagaccat tcatgttgtt gctcaggtcg cagacgtttt 4800
gcagcagcag tcgcttcacg ttcgctcgcg tatcggtgat tcattctgct aaccagtaag 4860
gcaaccccgc cagcctagcc gggtcctcaa cgacaggagc acgatcatgc gcacccgtgg 4920
ccaggaccca acgctgcccg aaatt                                       4945
```

<210> 4
<211> 4951
<212> ADN
<213> Séquence artificielle

<220>
<223> Description de la séquence artificielle: plasmide pMK84

<400> 4

```
ccgacaccat cgaatggcgc aaaacctttc gcggtatggc atgatagcgc ccggaagaga 60
gtcaattcag ggtggtgaat gtgaaaccag taacgttata cgatgtcgca gagtatgccg 120
gtgtctctta tcagaccgtt tcccgcgtgg tgaaccaggc cagccacgtt tctgcgaaaa 180
cgcgggaaaa agtggaagcg gcgatggcgg agctgaatta cattcccaac cgcgtggcac 240
aacaactggc gggcaaacag tcgttgctga ttggcgttgc cacctccagt ctggccctgc 300
acgcgccgtc gcaaattgtc gcggcgatta aatctcgcgc cgatcaactg ggtgccagcg 360
tggtggtgtc gatggtagaa cgaagcggcg tcgaagcctg taaagcggcg gtgcacaatc 420
ttctcgcgca acgcgtcagt gggctgatca ttaactatcc gctggatgac caggatgcca 480
ttgctgtgga agctgcctgc actaatgttc cggcgttatt tcttgatgtc tctgaccaga 540
cacccatcaa cagtattatt ttctcccatg aagacggtac gcgactgggc gtggagcatc 600
tggtcgcatt gggtcaccag caaatcgcgc tgttagcggg cccattaagt tctgtctcgg 660
cgcgtctgcg tctggctggc tggcataaat atctcactcg caatcaaatt cagccgatag 720
cggaacggga aggcgactgg agtgccatgt ccggttttca acaaaccatg caaatgctga 780
atgagggcat cgttcccact gcgatgctgg ttgccaacga tcagatggcg ctgggcgcaa 840
tgcgcgccat taccgagtcc gggctgcgcg ttggtgcgga tatctcggta gtgggatacg 900
acgataccga agacagctca tgttatatcc cgccgttaac caccatcaaa caggattttc 960
gcctgctggg gcaaaccagc gtggaccgct tgctgcaact ctctcagggc caggcggtga 1020
agggcaatca gctgttgccc gtctcactgg tgaaaagaaa aaccaccctg gcgcccaata 1080
cgcaaaccgc ctctccccgc gcgttggccg attcattaat gcagctggca cgacaggttt 1140
cccgactgga aagcgggcag tgagcgcaac gcaattaatg tgagttagct cactcattag 1200
gcacaattct catgtttgac agcttatcat cgactgcacg gtgcaccaat gcttctggcg 1260
```

```
tcaggcagcc atcggaagct gtggtatggc tgtgcaggtc gtaaatcact gcataattcg 1320
tgtcgctcaa ggcgcactcc cgttctggat aatgtttttt gcgccgacat cataacggtt 1380
ctggcaaata tttctgaaat gagctgttga caattaatca tcggctcgta taatgtgtgg 1440
aattgtgagc ggataacaat ttcacacagg aaacagaatt aataatgtat cgattaaata 1500
aggaggaata acatatgagg ggatcccacc atcaccatca ccacggtgga ggtggatctg 1560
gtggaggtgg atctaagaag aagaagaaga agggttctgt cgacgaatcc atggacgaat 1620
tcgagctcgg tacccggaga tctctcgagc tgcagcatgc aagcttcccg gggatctagt 1680
ctagactaga gcttagcttg gctgttttgg cggatgagag aagattttca gcctgataca 1740
gattaaatca gaacgcagaa gcggtctgat aaaacagaat ttgcctggcg gcagtagcgc 1800
ggtggtccca cctgacccca tgccgaactc agaagtgaaa cgccgtagcg ccgatggtag 1860
tgtggggtct ccccatgcga gagtagggaa ctgccaggca tcaaataaaa cgaaaggctc 1920
agtcgaaaga ctgggccttt cgttttatct gttgtttgtc ggtgaacgct ctcctgagta 1980
ggacaaatcc gccgggagcg gatttgaacg ttgcgaagca acggcccgga gggtggcggg 2040
caggacgccc gccataaact gccaggcatc aaattaagca gaaggccatc ctgacggatg 2100
gcctttttgc gtttctacaa actcttttgt ttattttttct aaatacattc aaatatgtat 2160
ccgctcatga gacaataacc ctgataaatg cttcaataat attgaaaaag gaagagtatg 2220
agtattcaac atttccgtgt cgcccttatt cccttttttg cggcattttg ccttcctgtt 2280
tttgctcacc cagaaacgct ggtgaaagta aaagatgctg aagatcagtt gggtgcacga 2340
gtgggttaca tcgaactgga tctcaacagc ggtaagatcc ttgagagttt tcgccccgaa 2400
gaacgttttc caatgatgag cacttttaaa gttctgctat gtggcgcggt attatcccgt 2460
gttgacgccg ggcaagagca actcggtcgc cgcatacact attctcagaa tgacttggtt 2520
gagtactcac cagtcacaga aaagcatctt acggatggca tgacagtaag agaattatgc 2580
agtgctgcca taaccatgag tgataacact gcggccaact tacttctgac aacgatcgga 2640
ggaccgaagg agctaaccgc ttttttgcac aacatggggg atcatgtaac tcgccttgat 2700
cgttgggaac cggagctgaa tgaagccata ccaaacgacg agcgtgacac cacgatgcct 2760
gtagcaatgg caacaacgtt gcgcaaacta ttaactggcg aactacttac tctagcttcc 2820
cggcaacaat taatagactg gatggaggcg gataaagttg caggaccact tctgcgctcg 2880
gcccttccgg ctggctggtt tattgctgat aaatctggag ccggtgagcg tgggtctcgc 2940
ggtatcattg cagcactggg gccagatggt aagccctccc gtatcgtagt tatctacacg 3000
acggggagtc aggcaactat ggatgaacga aatagacaga tcgctgagat aggtgcctca 3060
ctgattaagc attggtaact gtcagaccaa gtttactcat atatacttta gattgattta 3120
aaacttcatt tttaatttaa aaggatctag gtgaagatcc tttttgataa tctcatgacc 3180
aaaatccctt aacgtgagtt ttcgttccac tgagcgtcag accccgtaga aaagatcaaa 3240
ggatcttctt gagatccttt ttttctgcgc gtaatctgct gcttgcaaac aaaaaaacca 3300
ccgctaccag cggtggtttg tttgccggat caagagctac caactctttt tccgaaggta 3360
actggcttca gcagagcgca gataccaaat actgtccttc tagtgtagcc gtagttaggc 3420
caccacttca agaactctgt agcaccgcct acatacctcg ctctgctaat cctgttacca 3480
gtggctgctg ccagtggcga taagtcgtgt cttaccgggt tggactcaag acgatagtta 3540
ccggataagg cgcagcggtc gggctgaacg gggggttcgt gcacacagcc cagcttggag 3600
cgaacgacct acaccgaact gagataccta cagcgtgagc tatgagaaag cgccacgctt 3660
cccgaaggga gaaaggcgga caggtatccg gtaagcggca gggtcggaac aggagagcgc 3720
acgagggagc ttccaggggg aaacgcctgg tatctttata gtcctgtcgg gtttcgccac 3780
ctctgacttg agcgtcgatt tttgtgatgc tcgtcagggg ggcggagcct atggaaaaac 3840
gccagcaacg cggccttttt acggttcctg gccttttgct ggccttttgc tcacatgttc 3900
tttcctgcgt tatcccctga ttctgtggat aaccgtatta ccgcctttga gtgagctgat 3960
accgctcgcc gcagccgaac gaccgagcgc agcgagtcag tgagcgagga agcggaagag 4020
cgcctgatgc ggtattttct ccttacgcat ctgtgcggta tttcacaccg catatggtgc 4080
actctcagta caatctgctc tgatgccgca tagttaagcc agtatacact ccgctatcgc 4140
```

```
tacgtgactg ggtcatggct gcgccccgac acccgccaac acccgctgac gcgccctgac 4200
gggcttgtct gctcccggca tccgcttaca gacaagctgt gaccgtctcc gggagctgca 4260
tgtgtcagag gttttcaccg tcatcaccga aacgcgcgag gcagctgcgg taaagctcat 4320
cagcgtggtc gtgaagcgat tcacagatgt ctgcctgttc atccgcgtcc agctcgttga 4380
gtttctccag aagcgttaat gtctggcttc tgataaagcg ggccatgtta agggcggttt 4440
tttcctgttt ggtcacttga tgcctccgtg taagggggaa tttctgttca tgggggtaat 4500
gataccgatg aaacgagaga ggatgctcac gatacgggtt actgatgatg aacatgcccg 4560
gttactggaa cgttgtgagg gtaaacaact ggcggtatgg atgcggcggg accagagaaa 4620
aatcactcag ggtcaatgcc agcgcttcgt taatacagat gtaggtgttc cacagggtag 4680
ccagcagcat cctgcgatgc agatccggaa cataatggtg cagggcgctg acttccgcgt 4740
ttccagactt tacgaaacac ggaaaccgaa gaccattcat gttgttgctc aggtcgcaga 4800
cgttttgcag cagcagtcgc ttcacgttcg ctcgcgtatc ggtgattcat tctgctaacc 4860
agtaaggcaa ccccgccagc ctagccgggt cctcaacgac aggagcacga tcatgcgcac 4920
ccgtggccag gacccaacgc tgcccgaaat t                                4951
```

<210> 5
<211> 30
<212> ADN
<213> Séquence artificielle

<220>
<223> Description de la séquence artificielle: bras espaceur

<400> 5
aggcctctcg agatcgaagg tcgggtcgac 30

<210> 6
<211> 30
<212> ADN
<213> Séquence artificielle

<220>
<223> Description de la séquence artificielle: bras espaceur

<400> 6
ggtggaggtg gatctggtgg aggtggatct 30

<210> 7
<211> 60
<212> ADN
<213> Séquence artificielle

<220>
<223> Description de la séquence artificielle: bras espaceur

<400> 7
aggcctctcg agatcgaagg tcgggtcgac ggtggaggtg gatctggtgg aggtggatct 60

<210> 8
<211> 60
<212> ADN
<213> Séquence artificielle

<220>
<223> Description de la séquence artificielle: bras espaceur

<400> 8
ggtggaggtg gatctggtgg aggtggatct aggcctctcg agatcgaagg tcgggtcgac 60

<210> 9
<211> 10
<212> PRT
<213> Séquence artificielle

<220>
<223> Description de la séquence artificielle: séquence codée par le bras espaceur SEQ ID NO:5

<400> 9

```
Arg Pro Leu Glu Ile Glu Gly Arg Val Asp
 1               5                  10
```

<210> 10
<211> 10
<212> PRT
<213> Séquence artificielle

<220>
<223> Description de la séquence artificielle: séquence codée par le bras espaceur SEQ ID NO:6

<400> 10

```
Gly Gly Gly Gly Ser Gly Gly Gly Gly Ser
 1               5                  10
```

<210> 11
<211> 20
<212> PRT
<213> Séquence artificielle

<220>
<223> Description de la séquence artificielle: séquence codée par le bras espaceur SEQ ID NO:7

<400> 11

```
Arg Pro Leu Glu Ile Glu Gly Arg Val Asp Gly Gly Gly Gly Ser Gly
 1               5                  10                  15

Gly Gly Gly Ser
            20
```

<210> 12
<211> 20
<212> PRT

<213> Séquence artificielle

<220>
<223> Description de la séquence artificielle: séquence codée par le bras espaceur SEQ ID NO:8

<400> 12

```
Gly Gly Gly Gly Ser Gly Gly Gly Gly Ser Arg Pro Leu Glu Ile Glu
1               5                   10                  15

Gly Arg Val Asp
            20
```

<210> 13
<211> 231
<212> PRT
<213> p24 de VIH-1 (souche HXB2)

<400> 13

```
Pro Ile Val Gln Asn Ile Gln Gly Gln Met Val His Gln Ala Ile Ser
1               5                   10                  15

Pro Arg Thr Asn Leu Ala Trp Val Lys Val Val Glu Glu Lys Ala Phe
            20                  25                  30

Ser Pro Glu Val Ile Pro Met Phe Ser Ala Leu Ser Glu Gly Ala Thr
        35                  40                  45
```

```
Pro Gln Asp Leu Asn Thr Met Leu Asn Thr Val Gly Gly His Gln Ala
    50                  55                  60

Ala Met Gln Met Leu Lys Glu Thr Ile Asn Glu Glu Ala Ala Glu Trp
65                  70                  75                  80

Asp Arg Val His Pro Val His Ala Gly Pro Ile Ala Pro Gly Gln Met
                85                  90                  95

Arg Glu Pro Arg Gly Ser Asp Ile Ala Gly Thr Thr Ser Thr Leu Gln
            100                 105                 110

Glu Gln Ile Gly Trp Met Thr Asn Asn Pro Pro Ile Pro Val Gly Glu
        115                 120                 125

Ile Tyr Lys Arg Trp Ile Ile Leu Gly Leu Asn Lys Ile Val Arg Met
    130                 135                 140

Tyr Ser Pro Thr Ser Ile Leu Asp Ile Arg Gln Gly Pro Lys Glu Pro
145                 150                 155                 160

Phe Arg Asp Tyr Val Asp Arg Phe Tyr Lys Thr Leu Arg Ala Glu Gln
                165                 170                 175

Ala Ser Gln Glu Val Lys Asn Trp Met Thr Glu Thr Leu Leu Val Gln
            180                 185                 190

Asn Ala Asn Pro Asp Cys Lys Thr Ile Leu Lys Ala Leu Gly Pro Ala
        195                 200                 205

Ala Thr Leu Glu Glu Met Met Thr Ala Cys Gln Gly Val Gly Gly Pro
    210                 215                 220

Gly His Lys Ala Arg Val Leu
225                 230
```

<210> 14
<211> 243
<212> PRT
<213> p24 recombinante de VIH-1, RH24

<400> 14

Met Arg Gly Ser His His His His His His Gly Ser Val Asp Glu Ser
1 5 10 15

Met Val Gln Asn Ile Gln Gly Gln Met Val His Gln Ala Ile Ser Pro

```
                    20                  25                  30

Arg Thr Asn Leu Ala Trp Val Lys Val Val Glu Glu Lys Ala Phe Ser
        35                  40                  45

Pro Glu Val Ile Pro Met Phe Ser Ala Leu Ser Glu Gly Ala Thr Pro
    50                  55                  60

Gln Asp Leu Asn Thr Met Leu Asn Thr Val Gly Gly His Gln Ala Ala
65                  70                  75                  80

Met Gln Met Leu Lys Glu Thr Ile Asn Glu Glu Ala Ala Glu Trp Asp
                85                  90                  95

Arg Val His Pro Val His Ala Gly Pro Ile Ala Pro Gly Gln Met Arg
            100                 105                 110

Glu Pro Arg Gly Ser Asp Ile Ala Gly Thr Thr Ser Thr Leu Gln Glu
        115                 120                 125

Gln Ile Gly Trp Met Thr Asn Asn Pro Pro Ile Pro Val Gly Glu Ile
    130                 135                 140

Tyr Lys Arg Trp Ile Ile Leu Gly Leu Asn Lys Ile Val Arg Met Tyr
145                 150                 155                 160

Ser Pro Thr Ser Ile Leu Asp Ile Arg Gln Gly Pro Lys Glu Pro Phe
                165                 170                 175

Arg Asp Tyr Val Asp Arg Phe Tyr Lys Thr Leu Arg Ala Glu Gln Ala
            180                 185                 190

Ser Gln Glu Val Lys Asn Trp Met Thr Glu Thr Leu Leu Val Gln Asn
        195                 200                 205

Ala Asn Pro Asp Cys Lys Thr Ile Leu Lys Ala Leu Gly Pro Ala Ala
    210                 215                 220

Thr Leu Glu Glu Met Met Thr Ala Cys Gln Gly Val Gly Gly Pro Gly
225                 230                 235                 240

Asp Leu Val
```

<210> 15
<211> 241
<212> PRT
<213> p24 recombinante de VIH-1, R24H

<400> 15

```
Met Val Gln Asn Ile Gln Gly Gln Met Val His Gln Ala Ile Ser Pro
 1               5                  10                  15

Arg Thr Asn Leu Ala Trp Val Lys Val Val Glu Glu Lys Ala Phe Ser
            20                  25                  30

Pro Glu Val Ile Pro Met Phe Ser Ala Leu Ser Glu Gly Ala Thr Pro
        35                  40                  45

Gln Asp Leu Asn Thr Met Leu Asn Thr Val Gly Gly His Gln Ala Ala
    50                  55                  60

Met Gln Met Leu Lys Glu Thr Ile Asn Glu Glu Ala Ala Glu Trp Asp
65                  70                  75                  80

Arg Val His Pro Val His Ala Gly Pro Ile Ala Pro Gly Gln Met Arg
                85                  90                  95

Glu Pro Arg Gly Ser Asp Ile Ala Gly Thr Thr Ser Thr Leu Gln Glu
            100                 105                 110

Gln Ile Gly Trp Met Thr Asn Asn Pro Pro Ile Pro Val Gly Glu Ile
        115                 120                 125

Tyr Lys Arg Trp Ile Ile Leu Gly Leu Asn Lys Ile Val Arg Met Tyr
    130                 135                 140

Ser Pro Thr Ser Ile Leu Asp Ile Arg Gln Gly Pro Lys Glu Pro Phe
145                 150                 155                 160

Arg Asp Tyr Val Asp Arg Phe Tyr Lys Thr Leu Arg Ala Glu Gln Ala
                165                 170                 175

Ser Gln Glu Val Lys Asn Trp Met Thr Glu Thr Leu Leu Val Gln Asn
            180                 185                 190

Ala Asn Pro Asp Cys Lys Thr Ile Leu Lys Ala Leu Gly Pro Ala Ala
        195                 200                 205

Thr Leu Glu Glu Met Met Thr Ala Cys Gln Gly Val Gly Gly Pro Pro
    210                 215                 220

Leu Glu Ile Glu Gly Arg Val Asp His His His His His His Gly Ser
225                 230                 235                 240
```

Ile

<210> 16
<211> 252
<212> PRT
<213> p24 recombinante de VIH-1, RH24K

<400> 16

```
Met Arg Gly Ser His His His His His His Gly Ser Val Asp Glu Ser
1               5                   10                  15

Met Val Gln Asn Ile Gln Gly Gln Met Val His Gln Ala Ile Ser Pro
            20                  25                  30

Arg Thr Asn Leu Ala Trp Val Lys Val Val Glu Glu Lys Ala Phe Ser
        35                  40                  45

Pro Glu Val Ile Pro Met Phe Ser Ala Leu Ser Glu Gly Ala Thr Pro
    50                  55                  60

Gln Asp Leu Asn Thr Met Leu Asn Thr Val Gly Gly His Gln Ala Ala
65                  70                  75                  80

Met Gln Met Leu Lys Glu Thr Ile Asn Glu Glu Ala Ala Glu Trp Asp
                85                  90                  95

Arg Val His Pro Val His Ala Gly Pro Ile Ala Pro Gly Gln Met Arg
            100                 105                 110

Glu Pro Arg Gly Ser Asp Ile Ala Gly Thr Thr Ser Thr Leu Gln Glu
        115                 120                 125

Gln Ile Gly Trp Met Thr Asn Asn Pro Pro Ile Pro Val Gly Glu Ile
    130                 135                 140

Tyr Lys Arg Trp Ile Ile Leu Gly Leu Asn Lys Ile Val Arg Met Tyr
145                 150                 155                 160

Ser Pro Thr Ser Ile Leu Asp Ile Arg Gln Gly Pro Lys Glu Pro Phe
                165                 170                 175

Arg Asp Tyr Val Asp Arg Phe Tyr Lys Thr Leu Arg Ala Glu Gln Ala
            180                 185                 190

Ser Gln Glu Val Lys Asn Trp Met Thr Glu Thr Leu Leu Val Gln Asn
        195                 200                 205
```

```
Ala Asn Pro Asp Cys Lys Thr Ile Leu Lys Ala Leu Gly Pro Ala Ala
    210                 215                 220

Thr Leu Glu Glu Met Met Thr Ala Cys Gln Gly Val Gly Gly Pro Gly
225                 230                 235                 240

Lys Lys Lys Lys Lys Lys Ser Val Asp Glu Ser Leu
                245                 250
```

<210> 17
<211> 257
<212> PRT
<213> p24 recombinante de VIH-1, RK24H

<400> 17

```
Met Arg Gly Ser Lys Lys Lys Lys Lys Lys Gly Ser Val Asp Glu Ser
 1               5                  10                  15

Met Val Gln Asn Ile Gln Gly Gln Met Val His Gln Ala Ile Ser Pro
            20                  25                  30

Arg Thr Asn Leu Ala Trp Val Lys Val Val Glu Glu Lys Ala Phe Ser
        35                  40                  45

Pro Glu Val Ile Pro Met Phe Ser Ala Leu Ser Glu Gly Ala Thr Pro
    50                  55                  60

Gln Asp Leu Asn Thr Met Leu Asn Thr Val Gly Gly His Gln Ala Ala
65                  70                  75                  80

Met Gln Met Leu Lys Glu Thr Ile Asn Glu Glu Ala Ala Glu Trp Asp
                85                  90                  95

Arg Val His Pro Val His Ala Gly Pro Ile Ala Pro Gly Gln Met Arg
            100                 105                 110

Glu Pro Arg Gly Ser Asp Ile Ala Gly Thr Thr Ser Thr Leu Gln Glu
        115                 120                 125

Gln Ile Gly Trp Met Thr Asn Asn Pro Pro Ile Pro Val Gly Glu Ile
    130                 135                 140

Tyr Lys Arg Trp Ile Ile Leu Gly Leu Asn Lys Ile Val Arg Met Tyr
145                 150                 155                 160

Ser Pro Thr Ser Ile Leu Asp Ile Arg Gln Gly Pro Lys Glu Pro Phe
```

```
                165                 170                 175

Arg Asp Tyr Val Asp Arg Phe Tyr Lys Thr Leu Arg Ala Glu Gln Ala
            180                 185                 190

Ser Gln Glu Val Lys Asn Trp Met Thr Glu Thr Leu Leu Val Gln Asn
        195                 200                 205

Ala Asn Pro Asp Cys Lys Thr Ile Leu Lys Ala Leu Gly Pro Ala Ala
    210                 215                 220

Thr Leu Glu Glu Met Met Thr Ala Cys Gln Gly Val Gly Gly Pro Pro
225                 230                 235                 240

Leu Glu Ile Glu Gly Arg Val Asp His His His His His His Gly Ser
                245                 250                 255

Ile
```

<210> 18
<211> 249
<212> PRT
<213> p24 recombinante de VIH-1, R24KH

<400> 18

```
Met Val Gln Asn Ile Gln Gly Gln Met Val His Gln Ala Ile Ser Pro
1               5                   10                  15

Arg Thr Asn Leu Ala Trp Val Lys Val Val Glu Glu Lys Ala Phe Ser
            20                  25                  30

Pro Glu Val Ile Pro Met Phe Ser Ala Leu Ser Glu Gly Ala Thr Pro
        35                  40                  45

Gln Asp Leu Asn Thr Met Leu Asn Thr Val Gly Gly His Gln Ala Ala
    50                  55                  60

Met Gln Met Leu Lys Glu Thr Ile Asn Glu Glu Ala Ala Glu Trp Asp
65                  70                  75                  80

Arg Val His Pro Val His Ala Gly Pro Ile Ala Pro Gly Gln Met Arg
                85                  90                  95

Glu Pro Arg Gly Ser Asp Ile Ala Gly Thr Thr Ser Thr Leu Gln Glu
            100                 105                 110
```

```
Gln Ile Gly Trp Met Thr Asn Asn Pro Pro Ile Pro Val Gly Glu Ile
        115                 120                 125

Tyr Lys Arg Trp Ile Ile Leu Gly Leu Asn Lys Ile Val Arg Met Tyr
    130                 135                 140

Ser Pro Thr Ser Ile Leu Asp Ile Arg Gln Gly Pro Lys Glu Pro Phe
145                 150                 155                 160

Arg Asp Tyr Val Asp Arg Phe Tyr Lys Thr Leu Arg Ala Glu Gln Ala
                165                 170                 175

Ser Gln Glu Val Lys Asn Trp Met Thr Glu Thr Leu Leu Val Gln Asn
            180                 185                 190

Ala Asn Pro Asp Cys Lys Thr Ile Leu Lys Ala Leu Gly Pro Ala Ala
        195                 200                 205

Thr Leu Glu Glu Met Met Thr Ala Cys Gln Gly Val Gly Gly Pro Gly
    210                 215                 220

Lys Lys Lys Lys Lys Lys Arg Pro Leu Glu Ile Glu Gly Arg Val Asp
225                 230                 235                 240

His His His His His His Gly Ser Ile
                245
```

<210> 19
<211> 264
<212> PRT
<213> p24 recombinante de VIH-1, R24KsH

<400> 19

```
Met Val Gln Asn Ile Gln Gly Gln Met Val His Gln Ala Ile Ser Pro
 1               5                  10                  15

Arg Thr Asn Leu Ala Trp Val Lys Val Val Glu Glu Lys Ala Phe Ser
            20                  25                  30

Pro Glu Val Ile Pro Met Phe Ser Ala Leu Ser Glu Gly Ala Thr Pro
        35                  40                  45

Gln Asp Leu Asn Thr Met Leu Asn Thr Val Gly Gly His Gln Ala Ala
    50                  55                  60

Met Gln Met Leu Lys Glu Thr Ile Asn Glu Glu Ala Ala Glu Trp Asp
65                  70                  75                  80
```

Arg Val His Pro Val His Ala Gly Pro Ile Ala Pro Gly Gln Met Arg
85 90 95

Glu Pro Arg Gly Ser Asp Ile Ala Gly Thr Thr Ser Thr Leu Gln Glu
100 105 110

Gln Ile Gly Trp Met Thr Asn Asn Pro Pro Ile Pro Val Gly Glu Ile
115 120 125

Tyr Lys Arg Trp Ile Ile Leu Gly Leu Asn Lys Ile Val Arg Met Tyr
130 135 140

Ser Pro Thr Ser Ile Leu Asp Ile Arg Gln Gly Pro Lys Glu Pro Phe
145 150 155 160

Arg Asp Tyr Val Asp Arg Phe Tyr Lys Thr Leu Arg Ala Glu Gln Ala
165 170 175

Ser Gln Glu Val Lys Asn Trp Met Thr Glu Thr Leu Leu Val Gln Asn
180 185 190

Ala Asn Pro Asp Cys Lys Thr Ile Leu Lys Ala Leu Gly Pro Ala Ala
195 200 205

Thr Leu Glu Glu Met Met Thr Ala Cys Gln Gly Val Gly Gly Pro Gly
210 215 220

Lys Lys Lys Lys Lys Lys Arg Pro Leu Glu Ile Glu Gly Arg Val Asp
225 230 235 240

Gly Gly Gly Gly Ser Gly Gly Gly Gly Ser Gly Gly Gly Gly Ser His
245 250 255

His His His His His Gly Ser Ile
260

<210> 20
<211> 259
<212> PRT
<213> p24 recombinante de VIH-1, RHsK24

<400> 20

```
Met Arg Gly Ser His His His His His His Gly Gly Gly Gly Ser Gly
 1               5                  10                  15

Gly Gly Gly Ser Lys Lys Lys Lys Lys Lys Gly Ser Val Asp Glu Ser
```

20 25 30

Met Val Gln Asn Ile Gln Gly Gln Met Val His Gln Ala Ile Ser Pro
35 40 45

Arg Thr Asn Leu Ala Trp Val Lys Val Val Glu Glu Lys Ala Phe Ser
50 55 60

Pro Glu Val Ile Pro Met Phe Ser Ala Leu Ser Glu Gly Ala Thr Pro
65 70 75 80

Gln Asp Leu Asn Thr Met Leu Asn Thr Val Gly Gly His Gln Ala Ala
85 90 95

Met Gln Met Leu Lys Glu Thr Ile Asn Glu Glu Ala Ala Glu Trp Asp
100 105 110

Arg Val His Pro Val His Ala Gly Pro Ile Ala Pro Gly Gln Met Arg
115 120 125

Glu Pro Arg Gly Ser Asp Ile Ala Gly Thr Thr Ser Thr Leu Gln Glu
130 135 140

Gln Ile Gly Trp Met Thr Asn Asn Pro Pro Ile Pro Val Gly Glu Ile
145 150 155 160

Tyr Lys Arg Trp Ile Ile Leu Gly Leu Asn Lys Ile Val Arg Met Tyr
165 170 175

Ser Pro Thr Ser Ile Leu Asp Ile Arg Gln Gly Pro Lys Glu Pro Phe
180 185 190

Arg Asp Tyr Val Asp Arg Phe Tyr Lys Thr Leu Arg Ala Glu Gln Ala
195 200 205

Ser Gln Glu Val Lys Asn Trp Met Thr Glu Thr Leu Leu Val Gln Asn
210 215 220

Ala Asn Pro Asp Cys Lys Thr Ile Leu Lys Ala Leu Gly Pro Ala Ala
225 230 235 240

Thr Leu Glu Glu Met Met Thr Ala Cys Gln Gly Val Gly Gly Pro Gly
245 250 255

Asp Leu Val